# EUROPÄISCHE PATENTSCHRIFT

(11) **EP 2 421 582 B1**
(45) Veröffentlichungstag und Bekanntmachung des Hinweises auf die Patenterteilung: **23.11.2016**
(21) Anmeldenummer: 10714864.5
(22) Anmeldetag: 21.04.2010
(51) Int. Cl.: A61M 1/30, A61M 1/36, B29C 65/16, B29C 65/00, A61M 39/10, B29L 31/00

(54) **EXTERNE FUNKTIONSEINRICHTUNG UND SYSTEM**
EXTERNAL FUNCTIONAL DEVICE AND SYSTEM
DISPOSITIF FONCTIONNEL EXTERNE ET SYSTÈME

(30) Priorität: 23.04.2009 DE 102009018664; 10.06.2009 DE 102009024468; 10.06.2009 US 185643 P
(43) Veröffentlichungstag der Anmeldung: 29.02.2012
(73) Patentinhaber: Fresenius Medical Care Deutschland GmbH, 61352 Bad Homburg (DE)
(72) Erfinder: GRONAU, Sören, 64569 Nauheim (DE); GÜNTHER, Götz, 61440 Oberursel (DE); HÄCKER, Jürgen, 61267 Neu-Anspach (DE); LAUER, Martin, 66606 St. Wendel (DE); MANKE, Joachim, 35792 Löhnberg (DE); NIKOLIC, Dejan, 60599 Frankfurt (DE); WEIS, Manfred, 66606 St. Wendel (DE)
(74) Vertreter: Bobbert & Partner Patentanwälte PartmbB
(86) Internationale Anmeldenummer: PCT/EP2010/002488
(87) Internationale Veröffentlichungsnummer: WO 2010/121819

(56) Entgegenhaltungen:
- WO-A2-2008/011220
- DE-A1- 10 053 441
- DE-A1- 10 224 750
- US-A1- 2004 186 416
- US-A1- 2005 017 505

## Beschreibung

Die vorliegende Erfindung betrifft eine externe Funktionseinrichtung gemäß Anspruch 1. Sie betrifft ferner ein System gemäß dem Oberbegriff des Anspruchs 18.

Das Reinigen der bei Blutbehandlungen eingesetzten Apparaturen kann technisch aufwendig sein. Um eine ausreichende Hygiene bei akzeptablem Arbeitsaufwand gewährleisten zu können sowie aus anderen Gründen werden externe Funktionseinrichtungen, wie Blutkassetten eingesetzt. DE 102 24 750 A1 offenbart beispielsweise eine medizinische Flüssigkeitsbehandlungsvorrichtung mit einer in dieser einsetzbaren Kassette.

Eine solche Blutkassette kann dazu ausgelegt sein, möglichst viele Funktionen zur Vorbereitung und Durchführung von Blutbehandlungsverfahren zu erfüllen.

Aufgabe der vorliegenden Erfindung ist es, eine weitere externe Funktionseinrichtung, ausgestaltet als Blutbehandlungskassette, bereitzustellen. Ferner soll ein System bestehend aus einer Blutbehandlungsvorrichtung, welche eine solche externe Funktionseinrichtung aufweist oder zu deren Ansteuerung oder Betätigung ausgestaltet ist, vorgeschlagen werden.

Die erfindungsgemäße Aufgabe wird gelöst durch eine externe Funktionsvorrichtung mit den Merkmalen des Anspruchs 1.

Die externe Funktionseinrichtung weist wenigstens einen Gehäusekörper, wenigstens eine in den Gehäusekörper integrierte Kammer zum Aufnehmen medizinischer Fluide, wenigstens einen in den Gehäusekörper integrierten Kanal zum Aufnehmen und/oder Führen eines medizinischen Fluids und wenigstens eine in den Gehäusekörper ganz oder teilweise integrierte Ventileinrichtung zum Steuern oder Regeln eines die externe Funktionseinrichtung durchströmenden Fluids auf.

Der Begriff "Gehäusekörper", wie er hierin verwendet wird, bezeichnet einen dreidimensional ausgeformten Körper, der aus einem zum Einsatz in einem medizinischen Behandlungsverfahren, wie beispielsweise einem Blutbehandlungsverfahren, geeigneten Material, wie beispielsweise einem Kunststoffmaterial, gebildet ist. Der Gehäusekörper kann beispielsweise mit Hilfe eines Guss- oder Spritzgussverfahrens herstellbar sein.

Der Begriff "Kammer", wie er hierin verwendet wird, bezeichnet ein Volumen, welches zum Aufnehmen wenigstens eines medizinischen Fluids geeignet ist. Das Volumen kann ein geschlossener Raum sein oder von einem solchen umschrieben sein. Es kann aber auch ein offener Raum sein oder von einem solchen teilweise umgeben sein, und erst durch die Anwesenheit eines weiteren Körpers - einem anderen als dem Gehäusekörper - zu einen geschlossenen oder bis auf Zu- oder Ableitungen für das Fluid geschlossenen Raum werden.

Kammern können dazu ausgelegt und vorgesehen sein, um Ventile und/oder Sensoren oder dergleichen aufzunehmen.

Der Begriff "Kanal" oder "Leitung" wie er hierin verwendet wird, bezeichnet eine Einrichtung, die zum Aufnehmen und/oder Führen medizinischer Fluide, wie beispielsweise Blut, Heparin oder andere Medikamente, Kochsalzlösung, Substituat und dergleichen, geeignet ist.

Kanäle oder Leitungen können abschnittsweise als geschlossene und/oder halboffene Strukturen ausgestaltet sein. Sie können beispielsweise mittels einer Abdeckungseinrichtung an wenigstens einer offenen Seite verschließbar und so gegen zum Beispiel Komponenten einer Blutbehandlungsvorrichtung und/oder gegen die Atmosphäre abdichtbar ausgestaltet sein.

Der Begriff "Ventileinrichtung" wie er hierin verwendet wird, bezeichnet eine zum Steuern oder Regeln geeignete Einrichtung, welche den Durchlass bzw. Durchgang von Fluiden durch Kanäle bzw. Leitungen und/oder Kammern der externen Funktionseinrichtung kontrollieren kann. Ventileinrichtungen können mittels dafür vorgesehener Steuer- oder Regeleinrichtungen angesteuert werden. Eine Ansteuerung kann beispielsweise automatisiert erfolgen. Geeignete Steuer- oder Regeleinrichtungen können beispielsweise in oder an der Blutbehandlungsvorrichtung vorgesehen sein.

Vorteilhafte Weiterbildungen der erfindungsgemäßen externen Funktionseinrichtung sind jeweils Gegenstand der Unteransprüche.

Unter einer Funktionseinrichtung kann erfindungsgemäß eine Einrichtung verstanden werden, mittels welcher Funktionen wie Leiten von Fluid mittels Leitungen, Ventilen, Abfangen von Gerinnseln und/oder dergleichen möglich ist.

Unter einer externen Funktionseinrichtung kann eine Einrichtung zu verstehen sein, welche nicht dauerhafter Bestandteil einer Behandlungsvorrichtung ist. Eine externe Funktionseinrichtung wird vielmehr von extern mit der Behandlungsvorrichtung zum Zwecke einer Behandlung verbunden.

Die externe Funktionseinrichtung ist an wenigstens einer ihrer Oberflächen mit einer Abdeckungseinrichtung versehen, welche Teil wenigstens einer integrierten Ventileinrichtung ist.

Die Abdeckungseinrichtung ist wenigstens in einem Abschnitt mit dem Gehäusekörper kraft- und/oder form- und/oder stoffschlüssig verbunden. Die Abdeckungseinrichtung kann beispielsweise mittels einer umlaufenden Schweißnaht oder andersartigen umlaufenden Verbindung mit dem Gehäusekörper verbunden sein. Es können auch weitere nichtumlaufende oder punktförmige oder lokale Verschweißungen oder Verbindungen (z.B. Verklebungen oder Verpressungen) der Abdeckungseinrichtung mit dem Gehäusekörper vorgesehen sein.

In weiter bevorzugten Ausführungsformen ist die Abdeckungseinrichtung in bestimmten Bereichen beidseits von Strukturen (wenigstens einer Struktur) mit der externen Funktionseinrichtung, insbesondere mit dem Gehäusekörper, verbunden. Beidseits kann erfindungsgemäß als an wenigstens zwei Seiten der jeweiligen Struktur verstanden werden. Unter einer beidseitigen Verbindung kann eine wenigstens zweifache Verbindung im Bereich, insbesondere unmittelbaren Bereich, oder in der Umgebung, insbesondere der unmittelbaren Umgebung, der Struktur zu verstehen sein.

Zu diesen Strukturen zählen u.a. Fluidkanäle, Leitungen oder andere Elemente der externen Funktionseinrichtung. Hierzu zählen vorzugsweise solche Elemente, die in einem Querschnitt senkrecht zur Haupterstreckungsebene der Abdeckungseinrichtung offen ausgestaltet sind und/oder mittels der Abdeckung gegenüber einem Äußeren oder der Atmosphäre abgedeckt sind.

Die beidseitige Verbindung kann eine Verschweißung sein. Sie kann jeweils fluiddicht sein, z.B. derart, dass über den Fügebereich (Bereich, in welchem z.B. verklebt oder verschweißt wurde) der Verbindung kein Fluidaustauch, insbesondere kein Flüssigkeitsaustausch, erfolgen kann. Die beidseitige Verbindung kann für einzelne Fluidkanäle, Leitungen oder andere Elemente, z.B. in ausgewählten Bereichen der externen Funktionseinrichtung, vorgesehen sein; sie kann für eine Mehrzahl hiervon oder für alle Fluidkanäle, Leitungen oder andere Elemente vorgesehen sein.

Eine beidseitige Verbindung kann eine Verbindung sowohl links als auch rechts der entsprechenden Struktur sein. Sie kann sowohl oben als auch unten bezogen auf die Struktur vorgesehen sein oder dergleichen.

Eine beidseitige Verbindung kann eine, zwei oder mehrere Schweißnähte entlang des Rands oder Umfangs oder der Ersteckung mindestens einer Struktur oder eines Abschnitts hiervon sein.

Eine beidseitige Verbindung kann ganz oder in Abschnitten hiervon länglich oder lang gestreckt sein.

Mittels der beidseitigen Verbindung kann in bestimmten erfindungsgemäßen Ausführungsformen der zum Anpressen der externen Funktionseinrichtung erforderliche Aufwand auf vorteilhafte Weise verringert werden. Insbesondere können in manchen erfindungsgemäßen Ausführungsformen die Anforderungen an die Präzision, mit welcher die Anpressung der externen Funktionseinrichtung z.B. an eine Blutbehandlungsvorrichtung erfolgt, auf vorteilhafte Weise niedriger sein. In bestimmten erfindungsgemäßen Ausführungsformen kann auf vorteilhafte Weise aufgrund der beidseitigen Verbindung ein niedriger Anpressdruck genügen. In manchen erfindungsgemäßen Ausführungsformen kann auf vorteilhafte Weise eine zuverlässigere Funktion von Ventilen, die auf mittels der beidseitigen Verbindung überdeckte Kanäle wirken, erzielt werden.

Eine Abdeckungseinrichtung kann insbesondere eine Folie sein.

Die Folie kann vorzugsweise eine Kunststofffolie sein. Hierzu kann vorzugsweise jede dem Fachmann geeignet erscheinende Laser-schweißbare Folie in Betracht kommen.

In einer weiteren bevorzugten Ausführungsform kann die externe Funktionseinrichtung Anschlüsse zu ihrer Verbindung mit einem extrakorporalen Kreislauf in Fluidkommunikation aufweisen.

Die externe Funktionseinrichtung ist als Kassette zu einer bzw. für eine Blutbehandlung ausgestaltet.

Weiter bevorzugt kann die externe Funktionseinrichtung mittels zwei Konnektoren mit wenigstens einer - vorzugsweise zwei - peristaltischen Pumpe(n) in Fluidverbindung verbindbar sein. Rollenpumpen können geeignete peristaltische Pumpen sein.

Die externe Funktionseinrichtung kann wenigstens ein - vorzugsweise zwei - Pumpschlauchsegment(e) aufweisen bzw. zum Aufnehmen eines solchen ausgestaltet bzw. vorgesehen sein.

In einer weiteren bevorzugten Ausführungsform weist die externe Funktionseinrichtung wenigstens eine Ventileinrichtung auf, welche wenigstens einen Steg und einen Abschnitt der Abdeckungseinrichtung aufweist. Der Steg ist am Gehäusekörper ausgestaltet. Steg und Abdeckungseinrichtung sind angeordnet, um mittels eines auf einen Steg einwirkenden Aktors einer Blutbehandlungsvorrichtung zur Veränderung eines Fluiddurchflusses im Sinne eines Ventils betätigbar zu sein.

Ein solcher "Steg" kann eine in die externe Funktionseinrichtung integrierte bzw. von einer Oberfläche derselben in beliebige Richtung hervorstehende Komponente bezeichnen. Diese kann aus dem gleichen Material wie die externe Funktionseinrichtung gebildet sein. Ein Steg kann beispielsweise während der Herstellung der externen Funktionseinrichtung mittels Guss- oder Spritzgussverfahren ausgestaltet werden.

In einer weiteren bevorzugten Ausführungsform ist das Fluid, welches die externe Funktionseinrichtung in deren Gebrauch durchströmt, ein Substituat, Heparin oder ein anderer pharmakologischer Wirkstoff, Kochsalzlösung (insbesondere 0,9%ige NaCl-Lösung), Blut, Luft sowie Kombinationen derselben.

Die externe Funktionseinrichtung kann insbesondere an eine Blutbehandlungsvorrichtung ankoppelbar sein. Weiter bevorzugt kann sie ausgestaltet und vorgesehen sein, um mittels einer Aufnahmeeinrichtung der Blutbehandlungsvorrichtung an die Blutbehandlungsvorrichtung ankoppelbar zu sein. Die externe Funktionseinrichtung kann insbesondere mit der der Abdeckungseinrichtung zugewandten Oberfläche an die Behandlungsvorrichtung ankoppelbar sein.

In einer weiteren bevorzugten Ausführungsform kann die externe Funktionseinrichtung nach hinten in einem Neigungswinkel, vorzugsweise zwischen 5° und 11 °, insbesondere in einem Neigungswinkel von im Wesentlichen oder genau 8°, bezogen auf eine Vertikale an die Blutbehandlungsvorrichtung ankoppelbar sein. Die externe Funktionseinrichtung ist vorzugsweise in einem oberen Bereich hiervon (im Gebrauchszustand) nach hinten geneigt.

In einer weiteren bevorzugten Ausführungsform weist die externe Funktionseinrichtung wenigstens eine Substituat-Zugabestelle auf, welche ein Berührschutzelement und/oder einen Tropfschutz aufweist. Auch andere Abschnitte, insbesondere jeder andere Abschnitt, der externen Funktionseinrichtung können ein Berührschutzelement und/oder einen Tropfschutz aufweisen.

Eine Verschlussfunktion der Ports (eines, mancher oder aller Ports) der externen Funktionseinrichtung kann auch durch Septen oder Rückschlagventile realisiert werden.

Der Tropfschutz kann beispielsweise durch eine integrierte Verschlusshülse realisiert sein.

Der Tropfschutz kann bevorzugt dazu dienen, ein Heraustropfen oder Herauslaufen von Substituat oder Blut oder eines Gemisches von Substituat und Blut während des Ausbaus der externen Funktionseinrichtung aus der Aufnahmeeinrichtung der Blutbehandlungsvorrichtung zu verhindern. Auf diese Weise kann eine hygienische Handhabung der benutzten und unreinen externen Funktionseinrichtung auch außerhalb der Behandlungseinrichtung weiter gewährleistet sein.

Die externe Funktionseinrichtung gemäß der vorliegenden Erfindung kann zur Verwendung in einem Blutbehandlungsverfahren unter Verwendung eines Double-Needle-Zugangs oder eines Single-Needle-Zugangs geeignet sein.

Das Blut des Patienten wird vorzugsweise bereits während der Entnahmephase aus dem Patienten durch den Dialysator geleitet, dabei dialysiert, (vorzugsweise unmittelbar) nach Durchlaufen des Dialysators in der Single-Needle-Kammer gespeichert und von dort in der Rückgabephase dem Patienten zurückgegeben. Das Blut wird dabei in dem "frischen" Zustand dialysiert, in welchem es aus dem Patienten kommt. Damit kann sich das mittels der erfindungsgemäßen Kassette zur Blutbehandlung durchgeführte Verfahren vorteilhaft von jenen herkömmlichen Verfahren unterscheiden, bei welchen dem Patienten Blut entnommen, dies in einer separaten Single-Needle-Kammer gespeichert, anschließend dialysiert und über einen venösen Luftabscheider zurück an den Patienten gegeben wird.

Die externe Funktionseinrichtung kann vorzugsweise wenigstens eine Single-Needle-Kammer aufweisen, in welcher ein Blutschwallumleitungselement angeordnet ist.

Ein solches "Blutschwallumleitungselement" oder Blutschwallelement kann zum Erzielen einer Strömungsverlangsamung, zum Erzeugen einer Turbulenz und/oder Umleiten des in die Single-Needle-Kammer einströmenden Bluts oder zum Auslöschen des Impulses des Blutschwalls geeignet und vorgesehen sein. Ein solches Blutschwallumleitungselement kann insbesondere strömungsoptimiert ausgestaltet sein. Es kann beispielsweise in Form einer ellipsoiden oder runden Säule ausgestaltet sein, welche an wenigstens einem Abschnitt ihres Umfangs mit einer Wandung der Single-Needle-Kammer verbunden ist.

Ohne Blutschwallumleitungselement könnte ein durch das Phantomventil einströmender Blutschwall ggf. eine Fontäne bewirken. Dies könnte zu Schwappbewegungen des Flüssigkeitsspiegels und/oder zur Schaumbildung führen. Mittels des Blutschwallelements wird der Gesamtblutschwall in zwei kleinere Blutschwalle aufgeteilt, wodurch der Impuls des Gesamtblutschwalls ausgelöscht werden kann und vorteilhaft Fontänenbildung, Schwappbewegung und/oder Schaumbildung vermieden werden können.

Als Blutschwallumleitungselement kann vorzugsweise ein Blutschwallumleitungselement verwendet werden, wie es in der von der Anmelderin der vorliegenden Erfindung in der Patentanmeldung 10 2009 024 466.2 mit dem Titel *"Aufnahmeeinrichtung zum Aufnehmen von medizinischen Fluiden sowie externe Funktionseinrichtung und medizinische Behandlungsvorrichtung*", die am 10. Juni 2009 beim Deutschen Patent- und Markenamt eingereicht wurde, offenbart wurde.

Die externe Funktionseinrichtung der vorliegenden Erfindung weist wenigstens eine venöse Blutkammer auf.

Die Single-Needle-Kammer ist oberhalb, bezogen auf die Ausrichtung der externen Funktionseinrichtung während ihrer Verwendung, der venösen Blutkammer angeordnet.

Die externe Funktionseinrichtung der vorliegenden Erfindung weist ein Single-Needle-Blutventil auf, welches zwischen der venösen Blutkammer und der Single-Needle-Kammer angeordnet ist.

Die venöse Blutkammer kann mittels einer Querschnittsvejüngung des Gehäusekörpers in wenigstens einen oberen Raum und wenigstens einen unteren Raum aufgeteilt sein.

Der obere Raum und der untere Raum können in Fluidkommunikation bzw. -Verbindung miteinander stehen.

Der obere Raum kann ausgestaltet sein, um eine tangentiale Zuströmung von die externe Funktionseinrichtung durchströmenden Fluiden zuzulassen oder zu erzeugen. Der obere Raum kann einen Bereich zum Erzeugen einer stabilen Rotationsströmung der die externe Funktionseinrichtung durchströmenden Fluide aufweisen.

Der untere Raum kann einen Bereich aufweisen, der im Wesentlichen oder vollständig frei von Rotationsströmung der die externe Funktionseinrichtung durchströmenden Fluide ist.

Bevorzugt können Wandungen oder Wandabschnitte des oberen Raums und/oder des unteren Raums der venösen Blutkammer einer Neigung der externen Funktionseinrichtung gegen eine Vertikale der Blutbehandlungsvorrichtung angepasst sein. Dies kann in vorteilhafter Weise ein strömungsoptimiertes Fließen oder Strömen bzw. Durchströmen des Bluts durch die venöse Blutkammer und das Aufsteigen von möglicherweise im Blut befindlicher Luft zwecks Abscheidung ermöglichen.

Die venöse Blutkammer kann ausgestaltet sein und insbesondere eine Luftabscheiderwirkung haben, wie in der von der Anmelderin der vorliegenden Erfindung in der Patentanmeldung 10 2009 024 465.4 mit dem Titel *"Luftabscheider, externe Funktionseinrichtung, Blutkreislauf sowie Behandlungsvorrichtung",* die am 10. Juni 2009 beim Deutschen Patent- und Markenamt (DPMA) eingereicht wurde, offenbart wurde.

In einer weiteren bevorzugten Ausführungsform der externen Funktionseinrichtung ist der Gehäusekörper als Hartteil ausgestaltet.

Das Hartteil kann ein im Wesentlichen aus einem Stück und einem Werkstoff gefertigter Gehäusekörper sein. Dieser kann ein Spritzgussteil sein. Er kann eine Mindeststeifigkeit von mehr als 400 N/mm², bevorzugt 1200-1800 N/mm² (Biege-E-Modul) aufweisen.

In einer weiteren bevorzugten Ausgestaltung der externen Funktionseinrichtung als eine Blutbehandlungskassette kann in einer weiter bevorzugten Ausführungsform durch die Abdeckungseinrichtung oder Folie hindurch der Druck im extrakorporalen Blutkreislauf vor dem Dialysator gemessen werden.

Die externe *Funktionseinrichtung* kann bevorzugt ein Einmalartikel sein, welcher nach einmaligem Gebrauch entsorgt wird.

Die erfindungsgemäße Aufgabe wird auch gelöst durch ein System gemäß dem Anspruch 18.

Die Blutbehandlungsvorrichtung des Systems weist wenigstens eine externen Funktionseinrichtung gemäß der vorliegenden Erfindung auf.

Die Blutbehandlungsvorrichtung des Systems weist wenigstens eine Steuereinrichtung und/oder Aktoren und/oder Sensoren zum Ansteuern und/oder Betätigen der externen Funktionseinrichtung auf.

Die Steuereinrichtung kann als CPU oder Teil hiervon ausgestaltet sein.

Die Steuereinrichtung und/oder die Aktoren können beispielsweise zum Betreiben bzw. Ansteuern, d.h. beispielsweise Steuern oder Regeln, einer Ventileinrichtung geeignet und vorgesehen sein. Sie können an einer Position der Blutbehandlungsvorrichtung angeordnet sein, welche einer Ventileinrichtung der externen *Funktionseinrichtung* in ihrem angekoppelten Zustand gegenüberliegt.

Die Blutbehandlungsvorrichtung des Systems weist wenigstens eine Aufnahmeeinrichtung zum Aufnehmen wenigstens einer externen Funktionseinrichtung gemäß der vorliegenden Erfindung auf. Dabei weist die Aufnahmeeinrichtung eine Ankoppelfläche zum Ankoppeln der externen Funktionseinrichtung gemäß der vorliegenden Erfindung auf. Eine solche Ankoppelfläche kann beispielsweise um einen Winkel gegen eine Vertikale, bezogen auf die Ausrichtung der Blutbehandlungsvorrichtung während ihrer Verwendung, geneigt sein, insbesondere nach hinten. Ein solcher Winkel kann zwischen 5 und 11 °, insbesondere im Wesentlichen oder genau 8° betragen.

Die Blutbehandlungsvorrichtung kann zum Durchführen eines Verfahrens oder eines Verfahrensschrittes, wie sie im Folgenden sowie unter Bezugnahme auf die Figuren beschrieben sind, geeignet sein.

In einer weiteren Ausführungsform weist die Blutbehandlungsvorrichtung eine Steuerung - z.B. in Gestalt einer CPU - zum Ansteuern oder Regeln einer externen Funktionseinrichtung gemäß der vorliegenden Erfindung und/oder zum Durchführen eines Verfahrens bzw. eines Verfahrensschrittes, wie sie im Folgenden sowie unter Bezugnahme auf die Figuren beschrieben sind, auf.

Ferner kann die Blutbehandlungsvorrichtung wenigstens einen Aktor zum Betätigen einer externen Funktionseinrichtung gemäß der vorliegenden Erfindung oder eines Abschnitts hiervon zum Durchführen eines hierin beschriebenen Verfahrens bzw. eines Verfahrensschrittes aufweisen.

Die Blutbehandlungsvorrichtung kann insbesondere auch Sensoren als Informationsgeber aufweisen, wobei die Informationen der Steuereinheit als Signale zum Betätigen einer externen *Funktionseinrichtung* gemäß der vorliegenden Erfindung zum Durchführen eines hierin beschriebenen Verfahrens bzw. eines Verfahrensschrittes dienen.

Die Blutbehandlungsvorrichtung kann beispielsweise eine Dialysiereinrichtung sein.

Die Blutbehandlungsvorrichtung kann eine Steuerung aufweisen zum Messen eines im extrakorporalen Kreislauf oder Blutkreislauf der als Blutbehandlungskassette ausgestalteten erfindungsgemäßen externen *Funktionseinrichtung* vorliegenden Parameters, beispielsweise eines Drucks, eines Differenzdrucks und dergleichen.

Der Differenzdruck kann zwischen der arteriellen kassettenintegrierten Kammer und der venösen kassettenintegrierten Kammer gemessen werden. Der Differenzdruck kann als Maß für die blutseitige Druckdifferenz des Dialysators verwendet werden. Die Steuerung der Blutbehandlungsvorrichtung kann konfiguriert sein zum Berechnen dieser Differenz, ggf. zum Vergleichen der Druckdifferenz mit Referenzwerten (welche beispielsweise in der Steuerung oder einem Speicher hinterlegt sein können), und optional zum Ausgeben von Steuersignalen.

Hierbei kann vorteilhaft beispielsweise eine beginnende Verstopfung des Dialysators frühzeitig oder rechtzeitig erkannt werden. Es können Gegenmaßnahmen getroffen werden.

Diese können die Zugabe von Antikoagulantien, wie z.B. Heparin, z.B. über die kassettenintegrierten Zugabestellen umfassen oder hieraus bestehen.

Ferner kann z.B. die Prädilution erhöht werden. Es kann von Postdilution auf Prädilution umgeschaltet werden.

Die kassettenintegrierten Messstellen können ferner vorteilhaft eine Transmembrandruckmessung über die Dialysatormembran hinweg ermöglichen.

Dazu können vier Messstellen vorliegen, an denen mittels entsprechender Einrichtungen gemessen wird und deren Messergebnisse mittels geeigneter Einrichtungen ausgewertet werden: jeweils eine am Filtereingang und am Filterausgang, und zwar jeweils blutseitig und dialysatseitig.

In manchen erfindungsgemäßen Ausführungsformen ist die externe Funktionseinrichtung in bestimmten Abschnitten hiervon (zumindest auch) in einer Richtung senkrecht zur Ankoppelebene (oder zu einem Hauptabschnitt hiervon) stärker oder dicker als andere Abschnitte ausgestaltet. Die stärker oder dicker ausgestalteten Abschnitte, von denen es einen oder mehrere geben kann, dienen der Aufnahme von Messvorrichtungen wie z.B. optischen Messvorrichtungen, Ultraschallvorrichtungen, Temperaturmessvorrichtungen, und dergleichen.

Denselben Nutzen können Abschnitte der externen Funktionseinrichtung haben, welche nicht stärker oder dicker ausgestaltet sind, sondern welche sich vor allem in der Hauptersteckungsebene der externen Funktionseinrichtung erstrecken, vorzugsweise parallel zu einer Hauptankoppelebene der externen Funktionseinrichtung und/oder parallel zu einer Aktor-Sensor-Platte der Behandlungsvorrichtung. Diese die externe Funktionseinrichtung verlängernden Abschnitte (einer oder mehrere), können zugleich stärker oder dicker ausgestaltet sein; dies ist erfindungsgemäß allerdings nicht erforderlich.

Die Messvorrichtungen können ausgehend von einer Seite der Tür der Behandlungsvorrichtung, mit welcher die externe Funktionseinrichtung in bestimmten erfindungsgemäßen Ausführungsformen zu ihrem Gebrauch mit der Behandlungsvorrichtung verpresst und/oder abgedeckt wird, mit der Behandlungsvorrichtung verbunden werden oder sein.

Die Messvorrichtungen können ausgehend von einer Seite einer Aktor-Sensor-Platte der Behandlungsvorrichtung, mittels welcher in manchen erfindungsgemäßen Ausführungsformen eine Funktions- oder Signalverbindung zwischen externer Funktionseinrichtung und Behandlungsvorrichtung erzielt wird, mit der Behandlungsvorrichtung verbunden werden oder sein.

In solchen stärker oder dicker oder länger ausgestalteten Abschnitten angeordnete Messvorrichtungen können beispielsweise zum Messen von Zuständen innerhalb von zu- oder abführenden Fluidkanälen der externen Funktionsvorrichtung (insbesondere solche Fluidkanäle, die ein Fluid von der externen Funktionseinrichtung abführen oder dieser zuführen) dienen. Sie können in unmittelbarer Umgebung zu solchen Fluidkanälen angeordnet sein.

Alle oder manche der stärker oder dicker oder länger ausgestalteten Abschnitte liegen vorzugsweise in einem Randbereich der externen Funktionseinrichtung. Dies kann vorteilhaft eine einfache Verbindung zwischen Messvorrichtung, welche jeweils in einem der vorgenannten Abschnitte liegt, und der Behandlungseinrichtung ermöglichen. Ferner kann diese Anordnung in einem Randbereich eine leichte Zugänglichkeit ermöglichen.

Die externe Funktionseinrichtung kann in den oben genannten stärker oder dicker oder länger ausgestalteten Abschnitten oder an anderer Stelle Messstellen zur Ankoppelung von Detektoren wie z.B. optischen Detektoren aufweisen zum Erkennen von Leitung- oder Ventilleckagen. Solche Leckagen können z.B. im Bereich der Phantomventile, der Rückschlagventile, den zu- oder ableitenden Leitungen (z.B. zu oder von den Ventilen) oder dergleichen auftreten. Die Messstellen und/oder die, insbesondere optischen, Detektoren können an entsprechender Stelle angeordnet sein.

Die externe Funktionseinrichtung kann in manchen erfindungsgemäßen Ausführungsformen eine oder mehrere Zugabestellen aufweisen, welche jeweils wenigstens ein Septum aufweisen. Das Septum kann vorgesehen sein, um bei der Zugabe leicht penetriert zu werden, wobei es dabei aber vorteilhafter Weise dennoch Verschluss und somit Sicherheit und Dichtigkeit bietet.

Die Zugabestellen sind vorzugsweise in die externe Funktionseinrichtung integriert oder mit dieser integral hergestellt.

Die Zugabestellen können in einem Stirnbereich oder Randbereich der externen Funktionseinrichtung angeordnet sein. Diese Anordnung kann in manchen erfindungsgemäßen Ausfiihrungsformen vorteilhaft einen leichteren Zugang zu den Zugabestellen ermöglichen. In bestimmten erfindungsgemäßen Ausführungsformen gilt dies besonders für den Fall, dass die externe Funktionseinrichtung sowohl mittels ihrer Vorder- als auch ihrer Rückseite zur Ankoppelung mit einer Behandlungsvorrichtung in Kontakt steht (z.B. verpresst ist) und somit Vorder- als auch Rückseite für eine Zugabe über das Septum nur schwer oder mühevoll erreichbar sind. Es können sich somit in manchen Ausführungsformen ergonomische Voreile ergeben.

In weiter bevorzugten Ausführungsformen können Zufuhrleitungen derart an oder in der externen Funktionseinrichtung angeordnet sein, dass sich die Zufuhrleitung (ganz oder teilweise) oder eine Verbindungsstelle (wie beispielsweise ein Verbindungsport der Zufuhrleitung) zur Zufuhrleitung in einem oberen Bereich der externen Funktionseinrichtung befinden - vorzugsweise bezogen auf eine bestimmungsgemäße Stellung oder Anordnung der externen Funktionseinrichtung während ihres bestimmungsgemäßen Gebrauchs (z.B. in einem mit der Behandlungsvorrichtung verpressten Zustand).

Der obere Bereich kann ein Randbereich sein. Der obere Bereich kann ein Bereich oberhalb einer Ankoppelfläche oder eines Ankoppelbereichs sein.

Die Zufuhrleitung kann eine Leitung für ein Antikoagulationsmittel sein. Sie kann eine Heparinleitung sein. Eine zugehörige Spritzenpumpe für das Antikoagulationsmittel, z.B. Heparin, kann beim Gebrauch der externen *Funktionseinrichtung* oberhalb der externen Funktionseinrichtung oder deren Ankoppelebene angeordnet sein.

Vorteile, die hiermit in manchen erfindungsgemäßen Ausführungsformen zu erzielen sind, umfassen wiederum ergonomische Vorteile, ferner Vorteile, die mit einer kürzeren Zufuhrleitung einhergehen, bessere Zugänglichkeit der Verbindungsstelle, und mehr.

Unter Zufuhrleitungen können in manchen Ausführungsformen Leitungen verstanden werden, über welche während der Benutzung der externen *Funktionseinrichtung* bei einer Blutbehandlung dem extrakorporal strömenden Blut Fluide zugeführt werden können oder welche hierfür vorgesehen sind.

Die Blutbehandlungsvorrichtung kann eine Steuerung aufweisen zum Ansteuern der Kassettenventile. Die Steuerung kann vorzugsweise frei programmierbar zwischen der kassettenintegrierten Prä- und Postdilution umschalten. Sie kann vorzugsweise den Substituatstrom (Volumenstrom) verändern. Informationsgeber können insbesondere die vor und nach dem Dialysator angeordneten kassettenintegrierten Druckmessstellen sein.

Die Verfahren werden im Folgenden erläutert.

Das Blutbehandlungsverfahren wird im Folgenden exemplarisch als ein Dialysierverfahren, etwa eine Hämodiafiltration, angenommen. Die Blutbehandlungsvorrichtung ist beispielsweise eine Dialysiereinrichtung.
Die Dialysiereinrichtung weist einen extrakorporalen Kreislauf mit einem arteriellen und einem venösen Abschnitt auf. Die Dialysiereinrichtung weist ferner eine arterielle und eine venöse Patientenschlauchklemme auf.

Während der Dialyse ist ein Patient über einen Patientenzugang, wie beispielsweise eine Fistel, einen Shunt oder einen Katheter beispielsweise in Form eines Double-Needle-Zugangs oder Single-Needle-Zugangs, mit dem extrakorporalen Kreislauf verbunden.

Der extrakorporale Kreislauf kann eine Blutpumpe zum Fördern von Blut sowie eine Substituatpumpe zum Fördern von Substituat und entsprechende Pumpschlauchsegmente aufweisen.

Die Blutpumpe und die Substituatpumpe können als peristaltische Pumpen, beispielsweise als Rollenpumpen, ausgestaltet sein.

Eine "Förderrichtung" oder "Strömungsrichtung" des Bluts während einer Dialysebehandlung bezeichnet eine Richtung, in der das zu reinigende Blut üblicherweise gefördert wird. Insbesondere kann es eine Richtung bezeichnen, die vom Patienten über eine arterielle Nadel, einen arteriellen Abschnitt, eine Dialysiereinrichtung (in den Figuren von unten nach oben), einen venösen Abschnitt und über eine venöse Nadel zurück zum Patienten verläuft.

Eine gegen diese Förderrichtung erfolgende Fluidförderung (insbesondere von Blut und/oder von Substituat) wird als Förderung bzw. Strömung in Gegenrichtung bezeichnet.

Die Blutbehandlungsvorrichtung weist eine Dialysiereinrichtung mit einem Dialysierflüssigkeitseintritt und einem Dialysierflüssigkeitsaustritt auf, wobei die Dialysierflüssigkeit durch die Dialysiereinrichtung in Gegenrichtung zum Blut (in den Figuren von oben nach unten) gefördert werden kann.

Ein Substituat kann durch eine Substituat-Zugabestelle eingeleitet und durch ein Prädilutions-Zugabeventil und/oder ein Postdilutions-Zugabeventil in den extrakorporalen Kreislauf eingetragen werden.

Die Begriffe "Uhrzeigersinn" oder "gegen den Uhrzeigersinn" beziehen sich auf die Figuren. Eine Blutpumpe und eine Substituatpumpe fördern gewöhnlich gegen den Uhrzeigersinn, wie es der üblichen Förderrichtung während einer Dialysebehandlung entsprechen kann.

Beim **Primen oder Befüllen** können das Prädilutions-Zugabeventil, das Postdilutions-Zugabeventil und ein Single-Needle-Blutventil zunächst jeweils offenstehen.

Vorzugsweise sind auch die arterielle Patientenschlauchklemme und die venöse Patientenschlauchklemme offen.

Der arterielle Patientenanschluss und der venöse Patientenanschluss sind vorzugsweise miteinander verbunden.

Ein Hahn, welcher ein maschinenseitiges Ventil (auch bekannt als "Rinse-Port") ist, über welchen eine Fluidverbindung zwischen dem extrakorporalen Kreislauf und der Atmosphäre oder einem Auffangbehälter herstellbar ist und über welchen die externe Funktionseinrichtung durchströmende Fluide aus dem extrakorporalen Kreislauf austragbar sind, ist vorzugsweise ebenfalls geschlossen.

Über die Substituat-Zugabestelle wird Substituat zugegeben.

Die Substituatpumpe wird gestartet, sie wird vorzugsweise gegen einen Uhrzeigersinn (bezogen auf die Zeichenebene der angehängten Figuren) betrieben. Die Blutpumpe wird gestartet, vorzugsweise im Uhrzeigersinn.

Vorzugsweise wird Substituat bis an oder vor das Postdilutions-Zugabeventil gefördert. Das Postdilutions-Zugabeventil wird vorzugsweise bei Erreichen geschlossen.

Substituat wird durch das Prädilutions-Zugabeventil hindurch gefördert, vorzugsweise in Richtung Dialysiereinrichtung und/oder in Richtung zur Blutpumpe.

Dabei kann das Substituat, welches in Richtung der Dialysiereinrichtung strömt, die Dialysiereinrichtung und den venösen Abschnitt des extrakorporalen Kreislaufs durchströmen und in eine venöse Blutkammer der externen Funktionseinrichtung eintreten.

Das Substituat, welches in Richtung zur Blutpumpe strömt, kann das Pumpschlauchsegment, das in die Blutpumpe eingelegt ist, im Uhrzeigersinn durchströmen. Vorzugsweise durchströmt das Substituat ferner die Verbindung zwischen arteriellem und venösem Patientenanschluss, durchströmt einen Gerinnselfänger der externen Funktionseinrichtung und gelangt in die venöse Blutkammer.

Am oder im Gerinnselfänger kann der Druck im extrakorporalen Kreislauf mittels einer geeigneten Druckmesseinrichtung gemessen werden. Dies kann vorzugsweise durch eine Abdeckungseinrichtung der externen Funktionseinrichtung, z.B. als Blutbehandlungskassette ausgestaltet, erfolgen. Ist diese als Folie ausgestaltet, so kann die Messung durch sie hindurch bzw. über die Folie erfolgen. Auf diese Weise kann somit vorzugsweise der Druck im extrakorporalen Kreislauf insbesondere nach Durchlaufen des Dialysators gemessen werden.

Dabei ist es möglich, dass das Substituat, welches in Richtung Dialysiereinrichtung strömt mit jenem Substituat, welches in Richtung zur Blutpumpe strömt, in der venösen Blutkammer vermischt wird.

Ist die venöse Blutkammer gefüllt, so können vorzugsweise das Prädilutions-Zugabeventil 51 und das Single-Needle-Blutventil geschlossen werden oder automatisch schließen.

Die Substituatpumpe stoppt vorzugsweise oder wird gestoppt. Das Prädilutions-Zugabeventil, das Postdilutions-Zugabeventil und das Single-Needle-Blutventil werden vorzugsweise geschlossen.

Unter Betreiben der Blutpumpe wird Substituat durch den extrakorporalen Kreislauf gefördert. In der Single-Needle-Kammer liegt vorzugsweise kein Substituat oder nur eine geringe Menge Substituat vor.

Die online-Füllprozedur kann auch wie folgt ablaufen:
1. Konnektion des automatischen Substituat-Konnektors.
2. Arterieller und venöser Patientenschlauch werden mit einem Rinse-Port der Blutbehandlungsvorrichtung verbunden, z. B. mittels eines geeigneten Verbinders, mit dem für das Ende des einen Patientenschlauchs ein Zugang in den anderen Patientenschlauch geschaffen wird. Das Ende des anderen Patientenschlauchs dient als Ablaufleitung in den Rinse-Port. Der Verbinder kann sich alternativ in der arteriellen oder der venösen Patientenleitung befinden.
3. Die venöse Patientenschlauchklemme wird geschlossen, das Postdilutionsventil wird geöffnet, das Prädilutionsventil wird geschlossen.
4. Füllen der venösen Kammer mittels der Substituatpumpe durch das Postdilutionsventil. Dabei erfolgt eine Luftabscheidung durch das Single-Needle-Ventil.
5. Die Blutpumpe läuft vorwärts und saugt aus der venösen Kammer Substituat an.
6. Sinkt der Pegel in der venösen Kammer, so wird über das Postdilutionsventil nachgefördert, bis ein Überschreiten der vorgegebenen Füllhöhe vom Level-Detektor erkannt wird. Während dieses sich bei Bedarf wiederholenden Vorgangs läuft die Blutpumpe kontinuierlich weiter.
7. Entlüften des Gerinnselfängers " von unten": Alle drei Kassettenventile sind geschlossen. Die arterielle Klemme ist geöffnet und die venöse Klemme ist geschlossen. Der Rinse-Port ist geschlossen.
8. Die Blutpumpe läuft kurz rückwärts und fördert ein kleines Volumen. Dadurch wird venös ein Unterdruck und arteriell ein Überdruck im extrakorporalen Blutkreislauf erzeugt.
9. Öffnen der venösen Klemme solange, bis ein Druckausgleich stattgefunden hat.
10. Fortsetzen des Füllens des extrakorporalen Blutkreislaufs.
11. Spülen des gefüllten extrakorporalen Blutkreislaufs: Mittels des venösen Luftblasendetektors wird das Auftreten von Luftblasen detektiert. Sobald im Verlauf eines vorgegebenen Zeitintervalls keine Luftblasen oder nahezu keine Luftblasen detektiert wurden, gilt der extrakorporale Blutkreislauf als gefüllt.
12. Während des Spülens wird Substituat durch das Prädilutionsventil gefördert und durch den Rinse-Port ("Hahn") verworfen.
13. Dabei sind sowohl die arterielle als auch die venöse Klemme geöffnet. Die Blutpumpe läuft rückwärts und fördert einen Teil des Substituats in den Rinse-Port.

Alternativ zum Online- Füllen (das Substituat wird dabei online in der Dialysemaschine bereitgestellt) ist auch das Füllen mit einem externen Beutel mit Kochsalzlösung als Quelle für die Befüllflüssigkeit möglich. Dazu wird die arterielle Patientenleitung an den Beutel mit Kochsalzlösung angeschlossen. Die venöse Patientenleitung wird an einem sog. Waste-Bag als Senke für die gebrauchte Kochsalzlösung angeschlossen. Die Blutpumpe läuft vorwärts. Durch das Öffnen des Prädilutionsventils und des Postdilutionsventils kann auch die zwischen diesen beiden Ventilen befindliche Leitung befüllt werden.

Bei beiden Verfahren wird der Patient erst nach Erreichen einer vorgegebenen Spülmenge an den extrakorporalen Blutkreislauf angeschlossen.

Beim **Spülen oder Rinsing** sind zunächst vorzugsweise sowohl das Prädilutions-Zugabeventil, das Postdilutions-Zugabeventil als auch das Single-Needle-Blutventil geschlossen.

Die arterielle Patientenschlauchklemme und die venöse Patientenschlauchklemme sind zu Beginn vorzugsweise geöffnet.

Der arterielle Patientenanschluss und der venöse Patientenanschluss sind weiterhin miteinander verbunden.

Substituat wird unter Betreiben der Blutpumpe durch den extrakorporalen Kreislauf gefördert, wobei sich beispielsweise kein Substituat in der Single-Needle-Kammer befindet.

Das Prädilutions-Zugabeventil wird nun vorzugsweise geöffnet, ebenso der Hahn (ein maschinenseitiges Ventil bzw. ein "Rinse-Port").

Unter Betreiben der Substituatpumpe (vorzugsweise gegen den Uhrzeigersinn der Zeichenebene) und der Blutpumpe (vorzugsweise im Uhrzeigersinn) wird vorzugsweise Substituat kontinuierlich durch den extrakorporalen Kreislauf gefördert. Die Blutpumpe kann langsamer als die Substituatpumpe rotieren.

Das Substituat kann über eine Ablaufleitung aus dem extrakorporalen Kreislauf austreten und verworfen werden.

Zur Durchführung einer Dialyse wird ein Patient mit dem extrakorporalen Kreislauf konnektiert. Die Konnektion kann beispielsweise mit Hilfe eines Double-Needle-Zugangs oder mit Hilfe eines Single-Needle-Zugangs erfolgen.
In einer **ersten Variante eines Patientenanschlusses** können das Prädilutions-Zugabeventil, das Postdilutions-Zugabeventil und das Single-Needle-Blutventil bevorzugt geschlossen sein.

Ein Patient kann mittels eines Double-Needle-Zugangs über eine arterielle Nadel und eine venöse Nadel an den extrakorporalen Kreislauf konnektiert werden.

Die arterielle Patientenschlauchklemme und die venöse Patientenschlauchklemme können zunächst vorzugsweise geschlossen sein.

Dann kann die arterielle Patientenschlauchklemme geöffnet werden.

Die Blutpumpe kann betrieben werden (vorzugsweise gegen den Uhrzeigersinn) und vorzugsweise Blut durch die arterielle Nadel in den extrakorporalen Kreislauf fördern.

Substituat kann am Dialysierflüssigkeitsaustritt aus dem extrakorporalen Kreislauf ausgetragen und verworfen werden.

Wenn das zu reinigende Blut an einem Bluteintritt an der Dialysiereinrichtung ankommt, kann die arterielle Patientenschlauchklemme vorzugsweise geschlossen und die venöse Patientenschlauchklemme sodann geöffnet werden.

Die Blutpumpe kann gestoppt werden.

Nun kann vorzugsweise Blut durch die venöse Nadel in den extrakorporalen Kreislauf und durch den Gerinnselfänger in die venöse Blutkammer und durch eine venöse Filterleitung zu einem Blutaustritt aus der Dialysiereinrichtung strömen.

Alternativ kann ein Patient über einen Patientenanschluss gemäß einer zweiten Variante mit dem extrakorporalen Kreislauf konnektiert werden.

Zunächst werden das Prädilutions-Zugabeventil, das Postdilutions-Zugabeventil und das Single-Needle-Blutventil vorzugsweise geschlossen sein.

Der Patient kann beispielsweise wiederum mittels eines Double-Needle-Zugangs über eine arterielle Nadel und eine venöse Nadel an den extrakorporalen Kreislauf konnektiert werden.

Die Blutpumpe kann nun betrieben werden (vorzugsweise gegen den Uhrzeigersinn) und Blut durch die arterielle Nadel in den extrakorporalen Kreislauf fördern. Das Blut kann vorzugsweise die Dialysiereinrichtung und die externe Funktionseinrichtung durchströmen. Das Blut kann vorzugsweise über die venöse Nadel in den Patienten eintreten.

Die arterielle und die venöse Patientenschlauchklemme können vorzugsweise offen bleiben.

Bei einer **Dialysebehandlung** ohne Zugabe von Substituat können das Prädilutions-Zugabeventil und das Postdilutions-Zugabeventil geschlossen sein. Bei Durchführung einer Dialysebehandlung unter Einsatz eines Double-Needle-Zugangs kann das Single-Needle-Blutventil vorzugsweise geschlossen sein.

Ferner sind die arterielle Patientenschlauchklemme und die venöse Patientenschlauchklemme bevorzugt offen, so dass Blut den extrakorporalen Kreislauf kontinuierlich durchströmen kann.

Bevorzugt kann die Blutpumpe das Blut durch die arterielle Nadel in den extrakorporalen Kreislauf und durch die venöse Nadel zum Patienten zurück fördern.

Das Blut durchströmt die Dialysiereinrichtung, wo es in vorteilhafter Weise mittels der in Gegenrichtung zum Blut durch die Dialysiereinrichtung strömenden Dialysierflüssigkeit gereinigt werden kann.

Vorzugsweise können auch beide Schritte, nämlich das arterielle Ansaugen und das venöse Ansaugen - zumindest über einen Zeitraum - parallel erfolgen.

Der Begriff **"Online HDF Prädilution"** bezeichnet ein Dialysierverfahren, insbesondere eine Hämodiafiltration, bei der dem zu reinigenden Blut Substituat zugesetzt wird.

Vorzugsweise können bei diesem Verfahren ("Online HDF Prädilution") das Prädilutions-Zugabeventil offen, das Postdilutions-Zugabeventil und das Single-Needle-Blutventil dagegen geschlossen sein.

Die arterielle Patientenschlauchklemme und die venöse Patientenschlauchklemme können vorzugsweise geöffnet sein.

Die Blutpumpe (läuft vorzugsweise gegen den Uhrzeigersinn) kann Blut durch die arterielle Nadel in den extrakorporalen Kreislauf und durch die venöse Nadel zum Patienten bzw. in dessen Gefäßsystem zurück fördern, wie vorstehend in Bezug auf eine Dialysebehandlung bereits beschrieben ist.

Die Substituatpumpe (läuft vorzugsweise gegen den Uhrzeigersinn) kann Substituat fördern, das sich am bzw. ab dem Prädilutions-Zugabeventil im arteriellen Abschnitt des extrakorporalen Blutkreislaufs mit dem Blut vermischen kann.

Die Dialysierflüssigkeit kann, wie vorstehend beschrieben, vorzugsweise in Gegenrichtung zum Blut durch die Dialysiereinrichtung geleitet werden und zum Reinigen des die Dialysiereinrichtung ebenfalls durchströmenden Bluts eingesetzt werden.

Der Begriff **"Online HDF Postdilution"** bezeichnet ein Dialysierverfahren, insbesondere eine Hämodiafiltration, bei der dem gereinigten Blut Substituat zugesetzt wird.

Vorzugsweise können hierbei das Postdilutions-Zugabeventil offen, das Prädilutions-Zugabeventil sowie das Single-Needle-Blutventil dagegen geschlossen sein.
Der Prozessablauf entspricht im Wesentlichen dem Ablauf der vorstehend beschriebenen "Online HDF Prädilution", außer dass das Substituat an bzw. ab dem Postdilutions-Zugabeventil im venösen Abschnitt zu dem gereinigten Blut gegeben werden und sich mit diesem vermischen kann.

Eine so genannte **"Online HDF Mischdilution"** bezeichnet einen Prozess, bei welchem zwischen einem Prozess der "Online HDF Prädilution"; wie er vorstehend beschrieben ist, und einem Prozess der "Online HDF Postdilution", wie er vorstehend beschrieben ist, umgeschaltet werden kann.

Dabei kann jeder Teilprozess der Prädilution oder Postdilution für ein bestimmtes Intervall beibehalten werden. Jeder Teilprozess kann kontinuierlich wiederholt werden.

Der zeitliche Anteil der Prä- oder Postdilution kann in festem oder veränderlichem Verhältnis stehen und kann in Abhängigkeit einer Messgröße verändert werden.

Alternativ zu einer Blutbehandlung unter Verwenden eines Double-Needle-Zugangs zum Patienten, wie er vorstehend beschrieben ist, kann die Blutbehandlung unter Einsatz eines Single-Needle-Zugangs ("**Cassette Integrated Single Needle**") ausgeführt werden.

Ein Single-Needle-Zugang kann eine Y-förmige Verzweigung in den arteriellen Abschnitt und den venösen Abschnitt des extrakorporalen Kreislaufs aufweisen.

Das Prädilutions-Zugabeventil und das Postdilutions-Zugabeventil können geschlossen sein. Das Single-Needle-Blutventil kann dagegen vorzugsweise geöffnet sein. Auf diese Weise kann eine Fluidkommunikation zwischen der venösen Blutkammer und einer Single-Needle-Kammer möglich sein.

Die arterielle Patientenschlauchklemme und die venöse Patientenschlauchklemme können geschlossen sein.

Der Patient wird mit dem extrakorporalen Kreislauf konnektiert und die arterielle Patientenschlauchklemme kann bevorzugt geöffnet werden.

Die Blutpumpe kann Blut durch den extrakorporalen Kreislauf über die venöse Blutkammer vorzugsweise in die Single-Needle-Kammer der externen Funktionseinrichtung fördern.

Wenn die Single-Needle-Kammer im Wesentlichen oder vollständig gefüllt ist, kann die Blutpumpe bevorzugt gestoppt und die arterielle Patientenschlauchklemme geschlossen werden. Sodann kann die venöse Patientenschlauchklemme geöffnet werden.

Durch den venösen Abschnitt kann Blut in das Gefäßsystem des Patienten bzw. zum Patienten zurückströmen.

Sodann wird die arterielle Patientenschlauchklemme vorzugsweise geöffnet.

Der Vorgang kann so oft wie erforderlich und/oder gewünscht kontinuierlich oder in bestimmten Intervallen wiederholt werden.

Auch eine Kombination von Single-Needle-Behandlung mit Hämodiafiltration ist möglich.

Im Anschluss an ein Blutbehandlungsverfahren kann das nach der Behandlung im extrakorporalen Kreislauf befindliche Blut vorzugsweise in den Patienten rückgeführt werden.

Bei einer **ersten Variante der Blutrückführung** können das Prädilutions-Zugabeventil offen und das Postdilutions-Zugabeventil und das Single-Needle-Blutventil geschlossen sein.

Die arterielle Patientenschlauchklemme kann vorzugsweise geschlossen sein, die venöse Patientenschlauchklemme dagegen geöffnet.
Die arterielle Nadel und die venöse Nadel eines Double-Needle-Zugangs können vorzugsweise am Patienten verbleiben.

Die Substituatpumpe (läuft vorzugsweise gegen den Uhrzeigersinn) kann betrieben werden und vorzugsweise Substituat durch das Prädilutions-Zugabeventil in den extrakorporalen Kreislauf fördern.

Das Substituat kann die Dialysiereinrichtung und den venösen Abschnitt des extrakorporalen Kreislaufs durchströmen und dabei das Blut verdrängen. Kurz bevor das Substituat die venöse Nadel erreicht, kann die Substituatpumpe vorzugsweise gestoppt werden.

Sodann kann die venöse Patientenschlauchklemme geschlossen und die arterielle Patientenaschlauchklemme geöffnet werden.

Die Substituatpumpe (läuft vorzugsweise gegen den Uhrzeigersinn) und die Blutpumpe (läuft vorzugsweise im Uhrzeigersinn) können betrieben werden.

Das Substituat kann ab bzw. am Prädilutions-Zugabeventil in den arteriellen Abschnitt in Richtung des Patienten gefördert werden. Kurz vor Erreichen der arteriellen Nadel können beide Pumpen vorzugsweise gestoppt werden.

Die Rückgabe durch die arterielle und die venöse Nadel kann auch simultan erfolgen. Die Substituatpumpe läuft dann mit einer höheren Rate vorwärts und die Blutpumpe mit einer geringeren Rate rückwärts.

In einer **zweiten Ausführungsform Blutrückführungsprozesses** können das Prädilutions-Zugabeventil, das Postdilutions-Zugabeventil und das Single-Needle-Blutventil geschlossen sein.

Die arterielle Patientenschlauchklemme und die venöse Patientenschlauchklemme können vorzugsweise geschlossen sein.

Vorzugsweise können wenigstens ein Sensor/Detektor im arteriellen Teil des extrakorporalen Blutkreislaufs und wenigstens ein Sensor/Detektor im venösen Teil des extrakorporalen Blutkreislaufs vorgesehen sein.

Die arterielle Nadel kann vom Patienten und/oder die arterielle Patientenleitung kann von der arteriellen Nadel dekonnektiert werden. Die arterielle Patientenschlauchklemme und die venöse Patientenschlauchklemme werden vorzugsweise geöffnet.

Die Blutpumpe (läuft vorzugsweise gegen den Uhrzeigersinn) kann betrieben werden und Blut durch die arterielle Nadel in den extrakorporalen Kreislauf fördern.

Wenn der Sensor/Detektor im arteriellen Teil des extrakorporalen Blutkreislaufs das Auftreten von Luft erfasst, kann die Blutpumpe vorzugsweise solange weiter fördern, bis Blut am Prädilutions-Zugabeventil ankommt. Sodann kann die Blutpumpe vorzugsweise gestoppt werden.

Der arterielle Patientenanschluss kann geschlossen werden.

Die Substituatpumpe kann betrieben werden (läuft vorzugsweise gegen den Uhrzeigersinn).

Das Prädilutions-Zugabeventil kann geöffnet werden und die Substituatpumpe kann vorzugsweise Substituat durch das Prädilutions-Zugabeventil in den extrakorporalen Kreislauf fördern.

Das Substituat kann den extrakorporalen Kreislauf durchströmen und vorzugsweise das darin befindliche Blut verdrängen. Wenn der Sensor/Detektor der venösen Patientenschlauchklemme das Auftreten von Substituat erkennt, kann die Substituatpumpe vorzugsweise solange weiter Blut und Substituat durch die venöse Nadel in den Patienten fördern, bis alles Blut aus dem extrakorporalen Kreislauf in den Patienten rückgeführt wurde.

Schließlich können die venöse Nadel gezogen und die Pumpen vorzugsweise gestoppt werden.

Zum **Entleeren** können das Prädilutions-Zugabeventil und das Postdilutions-Zugabeventil offen, das Single-Needle-Blutventil dagegen geschlossen sein.

Die arterielle Patientenschlauchklemme und die venöse Patientenschlauchklemme können geschlossen sein.

Der arterielle Patientenanschluss und der venöse Patientenanschluss können vorzugsweise miteinander verbunden sein.

Zu Beginn des Prozesses können die arterielle Patientenschlauchklemme und die venöse Patientenschlauchklemme geöffnet werden.

An der bzw. über die Substituat-Zugabestelle kann vorzugsweise Substituat in den extrakorporalen Kreislauf eingeleitet werden. Zum Entleeren kann Luft gefördert werden.

Die Substituatpumpe kann betrieben werden (läuft gegen Uhrzeigersinn) und Substituat durch den extrakorporalen Kreislauf bis zum Prädilutions-Zugabeventil und zum Postdilutions-Zugabeventil fördern.

Verbrauchtes Substituat kann den extrakorporalen Kreislauf vorzugsweise am Dialysierflüssigkeitsaustritt verlassen und verworfen werden. Die Luft kann gefördert werden und dadurch das Substituat verdrängen.

Das Substituat kann einerseits durch das Prädilutions-Zugabeventil und zum anderen durch das Postdilutions-Zugabeventil in Richtung Dialysiereinrichtung gefördert werden.

Das Prädilutions-Zugabeventil kann geschlossen werden. Die Blutpumpe kann gestartet werden.

Mittels Betreiben der Blutpumpe (läuft vorzugsweise gegen Uhrzeigersinn) und der Substituatpumpe (läuft vorzugsweise gegen Uhrzeigersinn) kann Luft durch den extrakorporalen Kreislauf - beginnend stromaufwärts des Postdilutions-Zugabeventils - durch die venöse Blutkammer, den Gerinnselfänger, den venösen Abschnitt, den arteriellen Abschnitt in Richtung der Dialysiereinrichtung gefördert werden. Es kann den extrakorporalen Kreislauf vorzugsweise durch den Dialysierflüssigkeitsaustritt verlassen. Das gebrauchte Substituat kann verworfen werden.

In bevorzugten erfindungsgemäßen Ausführungsformen weist die externe Funktionseinrichtung wenigstens eine Zapfstelle auf, geeignet und vorgesehen zum Entnehmen eines Fluids, bei welchem es sich nicht um Blut oder nicht ausschließlich um Blut handelt, aus der externen Funktionseinrichtung während die externe Funktionseinrichtung mit einer Behandlungseinrichtung verbunden in Gebrauch ist.

In bevorzugten erfindungsgemäßen Ausführungsformen weist die externe Funktionseinrichtung wenigstens eine Schlauchfixierung zum Fixieren wenigstens eines Abschnitts wenigstens eines Schlauchs an der externen Funktionseinrichtung während deren Ankopplung an eine Behandlungsvorrichtung auf.

In bevorzugten erfindungsgemäßen Ausführungsformen ist das Fluid Teil der Gruppe, welche Substituat, Heparin, Blut, Kochsalzlösung, Luft sowie Kombinationen derselben umfasst.

In bevorzugten erfindungsgemäßen Ausführungsformen ist die externe Funktionseinrichtung in einem Neigungswinkel, insbesondere von im Wesentlichen oder genau 8°, bezogen auf eine Vertikale an die Blutbehandlungsvorrichtung ankoppelbar.

In bevorzugten erfindungsgemäßen Ausführungsformen weist die externe Funktionseinrichtung wenigstens eine Single-Needle-Kammer auf, in welcher ein Blutschwallumleitungselement angeordnet ist.

In bevorzugten erfindungsgemäßen Ausführungsformen sind Wandungen des oberen Raums und/oder des unteren Raums der venösen Blutkammer einer Neigung der externen Funktionseinrichtung gegen eine Vertikale der Blutbehandlungsvorrichtung angepasst.

In bevorzugten erfindungsgemäßen Ausführungsformen weisen Wandungen der venösen Blutkammer wenigstens eine Einbuchtung auf.

In bevorzugten erfindungsgemäßen Ausführungsformen ist der Gehäusekörper als Hartteil ausgestaltet.

In bevorzugten erfindungsgemäßen Ausführungsformen weist die externe Funktionseinrichtung einen Originalitätsschutz auf, der mittels der Ausgestaltung der Substituat-Zugabestelle verwirklicht ist.

In bevorzugten erfindungsgemäßen Ausführungsformen ist der Originalitätsschutz verwirklich mittels eines Berührschutzelements oder der Verschlusshülse der Substituat-Zugabestelle, basierend auf einer Veränderung deren Position innerhalb der oder bezogen auf die Kassette und/oder auf einer Veränderung ihrer Gestalt.

In bevorzugten erfindungsgemäßen Ausführungsformen weist die externe Funktionseinrichtung einen Wiederverwendungsschutz auf, der mittels der Ausgestaltung der Substituat-Zugabestelle verwirklicht ist.

Dabei wird in bevorzugten erfindungsgemäßen Ausführungsformen eine Verschlusshülse für eine Wiederverwendung unbrauchbar gemacht.

In bevorzugten erfindungsgemäßen Ausführungsformen ist die Ankoppelfläche um einen Winkel gegen eine Vertikale, bezogen auf die Ausrichtung der Blutbehandlungsvorrichtung während ihrer Verwendung oder den Erdmittelpunkt, geneigt. In bevorzugten erfindungsgemäßen Ausführungsformen ist der Winkel gegen eine Vertikale, bezogen auf die Ausrichtung der Blutbehandlungsvorrichtung während ihrer Verwendung oder den Erdmittelpunkt zwischen 5 und 11°, insbesondere im Wesentlichen oder genau 8°.

In bevorzugten erfindungsgemäßen Ausführungsformen weist die Blutbehandlungsvorrichtung eine Steuerung zum Ansteuern oder Regeln einer erfindungsgemäßen externen Funktionseinrichtung.

In bevorzugten erfindungsgemäßen Ausführungsformen weist die erfindungsgemäße Blutbehandlungsvorrichtung wenigstens einen Aktor zum Betätigen einer erfindungsgemäßen externen Funktionseinrichtung oder eines Abschnitts hiervon zum Durchführen eines hierin beschriebenen Verfahrens auf.

In bevorzugten erfindungsgemäßen Ausführungsformen weist die Blutbehandlungsvorrichtung Sensoren zum Ausgeben von Signalen als Grundlage zum Betätigen einer erfindungsgemäßen externen Funktionseinrichtung zum Durchführen eines hierin beschriebenen Verfahrens auf.

Im Folgenden wird die vorliegende Erfindung anhand bevorzugter Ausführungsformen derselben unter Bezugnahme auf die Zeichnung beschrieben. In den Figuren der Zeichnung bezeichnen gleiche Bezugszeichen gleiche oder identische Elemente. Es gilt:
- Fig. 1: zeigt eine Seitenansicht einer an ihrer Vorderseite mit einer Abdeckungseinrichtung versehenen erfindungsgemäßen externen Funktionseinrichtung gemäß einer bevorzugten Ausführungsform;
- Fig. 2: zeigt die externe Funktionseinrichtung der Fig. 1 mit nach zerstörendem Aufschneiden aufgeklappter Abdeckungseinrichtung;
- Fig. 3: zeigt die externe Funktionseinrichtung aus Fig. 1 und Fig. 2 von ihrer Rückseite;
- Fig. 4: zeigt schematisch vereinfacht eine Phase während der Durchführung eines Vorbereitungs- oder Priming-Prozesses vor Ausführung eines Blutbehandlungsprozesses;
- Fig. 5: zeigt schematisch vereinfacht eine Phase während der Durchführung eines Spülungs- oder Rinsing-Prozesses vor Ausführung eines Blutbehandlungsprozesses;
- Fig. 6: zeigt schematisch vereinfacht eine Phase während der Durchführung eines ersten Prozesses zum Anschließen eines Patienten an einen extrakorporalen Kreislauf einer Blutbehandlungsvorrichtung;
- Fig. 7: zeigt schematisch vereinfacht eine Phase während der Durchführung eines zweiten Prozesses zum Anschließen bzw. Konnektieren eines Patienten an einen bzw. mit einem extrakorporalen Kreislauf einer Blutbehandlungsvorrichtung;
- Fig. 8: zeigt schematisch vereinfacht eine Phase während der Durchführung eines ersten Blutbehandlungsprozesses;
- Fig. 9: zeigt schematisch vereinfacht eine Phase während der Durchführung des Blutbehandlungsprozesses aus Fig. 8 unter Einsatz von Prädilution;
- Fig. 10: zeigt schematisch vereinfacht eine Phase während der Durchführung des Blutbehandlungsprozesses aus Fig. 8 unter Einsatz von Postdilution;
- Fig. 11: zeigt schematisch vereinfacht eine Phase während der Durchführung des Blutbehandlungsprozesses aus Fig. 8 unter Einsatz von Mischdilution (Prädilution);
- Fig. 12: zeigt schematisch vereinfacht eine Phase während der Durchführung des Blutbehandlungsprozesses aus Fig. 8 unter Einsatz von Mischdilution (Postdilution);
- Fig. 13: zeigt schematisch vereinfacht eine Phase während der Durchführung eines zweiten Blutbehandlungsprozesses unter Verwenden eines Single-Needle-Zugangs;
- Fig. 14: zeigt schematisch vereinfacht eine Phase während der Durchführung eines ersten Blutrückführungsprozesses;
- Fig. 15: zeigt schematisch vereinfacht eine Phase während der Durchführung eines zweiten Blutrückführungsprozesses;
- Fig. 16: zeigt schematisch vereinfacht eine Phase während der Durchführung eines Entleerungs- oder Emptying-Prozesses nach Ausführung eines Blutbehandlungsprozesses;
- Fig. 17: zeigt schematisch vereinfacht eine erfindungsgemäße externe Funktionseinrichtung in einer weiteren Ausführungsform, betrachtet von ihrer Vorderseite;
- Fig. 18: zeigt schematisch vereinfacht ein Detail aus der Darstellung der Fig. 17;
- Fig. 19: zeigt schematisch vereinfacht die erfindungsgemäße externe Funktionseinrichtung der weiteren Ausführungsform der Fig. 17, dargestellt in einer perspektivischen Ansicht mit Blick auf ihre Rückseite;
- Fig. 20: zeigt schematisch vereinfacht ein Detail der Darstellung der Fig. 19;
- Fig. 21: zeigt schematisch vereinfacht die erfindungsgemäße externe Funktionseinrichtung in einer wiederum weiteren Ausführungsform in einer Ansicht von ihrer Vorderseite;
- Fig. 22: zeigt schematisch vereinfacht ein Detail aus der Darstellung der Fig. 21;
- Fig. 23: zeigt schematisch vereinfacht ein weiteres Detail aus der Darstellung der Fig. 21;
- Fig. 24: zeigt schematisch vereinfacht eine erfindungsgemäße Ausführungsform der externen Funktionseinrichtung;
- Fig. 25: zeigt schematisch vereinfacht ein Detail aus der Darstellung der Fig. 24; und
- Fig. 26: zeigt schematisch vereinfacht ein weiteres Detail aus der Darstellung der Fig. 24.

Zur beispielhaften Erläuterung der vorliegenden Erfindung sind als Behandlungsvorrichtung eine Blutbehandlungsvorrichtung und als Verfahren ein Blutbehandlungsverfahren ausgewählt.

Die normalen Pfeile in den Figuren zeigen die Richtung des Blutstroms an. Die Blockpfeile zeigen jeweils die Richtung des Substituatstroms an. Die Doppelblockpfeile zeigen jeweils die Richtung des Dialysierflüssigkeitsstroms an.

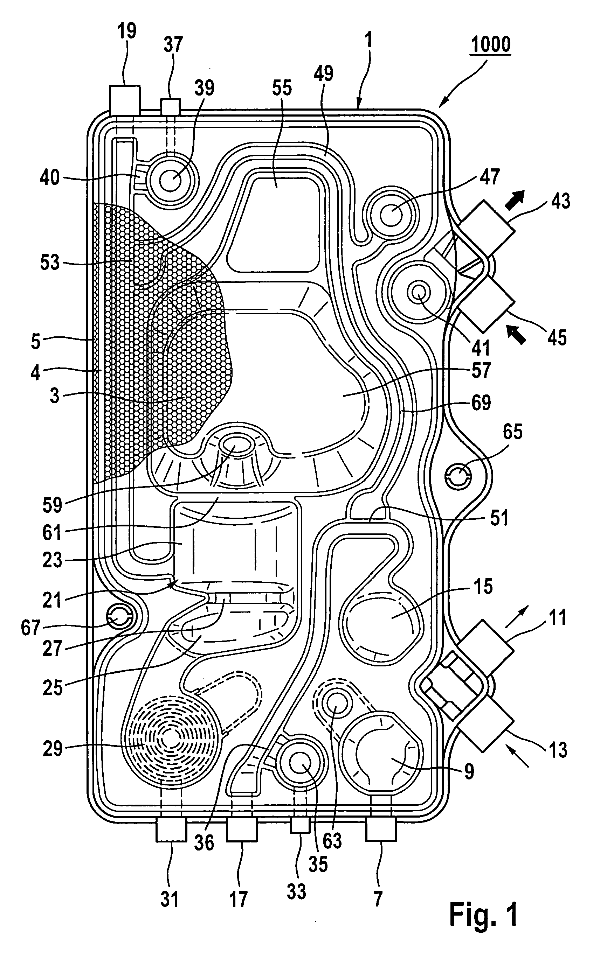

Fig. 1 zeigt eine Seitenansicht einer externen Funktionseinrichtung, welche an der Oberfläche, auf die in Fig. 1 geblickt wird, mit einer Abdeckungseinrichtung versehen ist.

Die externe Funktionseinrichtung ist hier exemplarisch als Kassette 1000 ausgestaltet.

Die Kassette 1000 weist ein Hartteil 1 auf. Wie in Fig. 1 exemplarisch gezeigt, weist das Hartteil 1 Kammern, Kanäle und Ventile auf. Wie in Fig. 1 exemplarisch weiter gezeigt ist, sind die Kammern, Kanäle und Ventile in das Hartteil 1 integriert bzw. zumindest teilweise vom Hartteil 1 ausgestaltet.
Die Kassette 1000 der Fig. 1 ist an ihrer Vorderseite mit einer Abdeckungseinrichtung, hier beispielsweise einer Folie 3, versehen. Die Abdeckungseinrichtung kann eben, d.h. plan, auf das Hartteil 1 aufgeschweißt sein.

Eine Ausgestaltung mit einer dreidimensionalen Ausführung der Schweiß- und Dichtkontur ist erfindungsgemäß ebenfalls möglich.

Die Abdeckungseinrichtung kann die Kammern und/oder Kanäle des Hartteils 1 der Kassette 1000 verschließen, und zwar gegenüber einer dem Hartteil 1 abgewandten Seite der Abdeckungseinrichtung und/oder gegenüber der Atmosphäre.

Wie in Fig. 1 zu erkennen ist, liegt die Folie an einem umlaufenden Dichtsteg 4 auf dem Hartteil 1 der Kassette 1000 auf. Die Folie 3 ist an einer umlaufenden Schweißnaht 5 mit dem Hartteil 1 der Kassette 1000 verschweißt.

Der umlaufende Dichtsteg kann alternativ freiliegend ausgeführt werden.

Die Folie 3 kann an weiteren lokalen Verschweißungen (nicht gezeigt) mit dem Hartteil 1 der Kassette 1000 verbunden sein. Diese können ebenfalls umlaufend, also geschlossen im Sinne einer abschließenden Begrenzung ähnlich einem Ring, und/oder punktförmig sein.

Die Folie 3 kann an lokalen Stellen punkt- oder linienförmig mit dem Hartteil der Kassette 1000 verbunden, z. B. verschweißt, sein, insbesondere an den Randzonen der flüssigkeitsführenden Kanäle.

Die Folie 3 kann durch Laser-Schweißen mit dem Hartteil der Kassette 1000 verbunden werden. Dabei ist es vorteilhaft, wenn der lokale Hitzeeintrag unter Verwendung von Licht absorbierender Komponenten erfolgt. Die Licht absorbierende Komponente kann Bestandteil des Materials der Folie und/oder des Hartteils sein oder eine Schicht, die zwischen Folie und Hartteil oder über der Folie angeordnet ist. Die Schicht kann eine Folienschicht sein.

Die Kassette 1000 kann wenigstens mit der in Fig. 1 gezeigten Vorderseite an eine Blutbehandlungsvorrichtung (in Fig. 1 nicht gezeigt) angekoppelt werden. Eine beispielhafte Vorgehensweise zum geeigneten Ankoppeln einer Kassette 1 an eine Ankoppelfläche einer Blutbehandlungsvorrichtung ist in den Patentanmeldungen 10 2009 012 633.3 mit dem Titel *"Vorrichtung zum Verbinden einer externen Funktionseinrichtung mit einer Anordnung, Anordnung aufweisend eine solche Vorrichtung und Verfahren zum Verbinden",* die am 10. März 2009 beim DPMA eingereicht wurde, und 10 2009 012 632.5 mit dem Titel *"Abdichtungseinrichtung zum Abdichten eines Volumens einer medizinischen Behandlungsanordnung gegen ein weiteres Volumen sowie Unordnung und Verfahren",* die ebenfalls am 10. März 2009 beim DPMA eingereicht wurde, beschrieben.

Die Kassette 1000 kann mit der Ebene der Folie 3 - oder über diese - an eine Ankoppelfläche der Blutbehandlungsvorrichtung angekoppelt werden. Die Ankoppelfläche kann vorzugsweise dreidimensional ausgeführt sein.

Die Ankoppelfläche der Blutbehandlungsvorrichtung kann zum Beispiel an einem in Fig. 1 oberen Abschnitt hiervon um 8° gegen eine in Fig. 1 von oben nach unten verlaufende Senkrechte nach hinten (in der Fig. 1 die sich vom Betrachter in die Zeichenebene hinein erstreckende Richtung) geneigt sein.

Die Kassette 1000 weist einen arteriellen Patientenanschluss 7 auf.

Die Kassette 1000 weist eine arterielle Druckmesskammer 9 auf. Diese kann entsprechende Sensoren aufweisen. Die Sensoren können bevorzugt über eine Verkabelung Signale übermitteln. Die Sensoren können aber auch so ausgeführt sein, dass sie kabellos Signale übermitteln.

Die Kassette 1000 weist einen Konnektor 11 für den Blutaustritt aus der Kassette 1000 sowie einen Konnektor 13 für den Bluteintritt in die Kassette 1000 auf.
Die beiden Konnektoren 11 und 13 sind mit einem Pumpschlauchsegment oder -set einer Blutpumpe verbindbar.

Die Kassette 1000 weist ferner eine Kammer 15 mit einer Druckmessstelle zur Druckmessung im extrakorporalen Blutkreislauf vor dem Dialysator ("Prä-Filter") bzw. nach der Pumpe ("Post-Pumpe") auf.

An der Kammer 15 kann durch die Folie 3 hindurch bzw. über die Folie 3 der Druck im extrakorporalen Kreislauf vor dem Dialysator gemessen werden.

Die Kassette 1000 weist eine arterielle Filterleitung 17 sowie eine venöse Filterleitung 19 auf.

Das Innere der Kassette 1000 weist eine venöse Blutkammer 21 auf. Die venöse Blutkammer 21 ist einen oberen Raum 23 und einen unteren Raum 25 unterteilt.

Der obere Raum 23 der venösen Blutkammer 21 kann eine seitlich tangentiale Bluteinströmung zulassen. Dabei kann Blut seitlich durch den Einlass (der linken Seite in Fig. 1) in den oberen Raum 23 einströmen und sich tangential zu den Wänden des oberen Raums 23 ausbreiten. Eine seitlich tangentiale Bluteinströmung kann eine Zone mit im Wesentlichen oder vollständig stabiler Rotationsströmung des Bluts in dem oberen Raum 23 der venösen Blutkammer 21 erzeugen.

Der untere Raum 25 der venösen Blutkammer 21 kann eine Beruhigungszone für die Blutströmung darstellen. Es ist möglich, dass in einer derartigen Beruhigungszone im Wesentlichen keine oder überhaupt keine Rotationsströmung des Bluts vorliegt.

Die venöse Blutkammer 21 ist durch eine Querschnittsverjüngung 27 des Hartteils 1 der Kassette 1000 in den oberen Raum 23 und den unteren Raum 25 aufgeteilt. Die Querschnittsverjüngung 27 reduziert den Querschnitt der venösen Blutkammer 21 derart in seiner Breite und Tiefe, dass sich eine Strömungsschnelle ergibt, nach deren Durchquerung ein die venöse Blutkammer 21 der Kassette 1000 durchströmendes Fluid eine langsamere Strömungsgeschwindigkeit annimmt. Der obere Raum 23 und der untere Raum 25 stehen in Fluidkommunikation.

Durch eine derartige Konstruktion, d.h. einer Aufteilung der venösen Blutkammer 21 in eine Zone mit im Wesentlichen oder vollständig stabiler Rotationsströmung des Bluts und in eine Beruhigungszone für die Blutströmung, kann vorteilhaft eine effiziente Abscheidung für Luft aus dem Blut oder Fluid erreicht werden.

Wandungen des oberen Raums 23 und des unteren Raums 25 der venösen Blutkammer 21 können in geeigneter Weise einer Neigung des oberen Abschnitts der Kassette 1000 in Fig. 1 gegen die Vertikale, beispielsweise einer Neigung des oberen Teils der in Fig. 1 gezeigten Kassette 1000 um 8° nach hinten (in die Zeichenebene hinein), angepasst werden. Sie können in geeigneter Weise derart gerundet ausgestaltet sein, dass sie vorteilhaft eine strömungsoptimierte Berührfläche für Fluide darstellt, welche die venöse Blutkammer 21 durchströmen.

Die Kassette 1000 weist einen Gerinnselfänger 29 auf.

Als Gerinnselfänger kann vorzugsweise ein Gerinnselfänger verwendet werden, wie er in der von der Anmelderin der vorliegenden Erfindung in der Patentanmeldung 10 2009 024 495.6 mit dem Titel *"Gerinnselfänger, externe Funktionseinrichtung, Blutkreislauf sowie Behandlarngs-vorrichtung",* die am 10. Juni 2009 beim DPMA eingereicht wurde, offenbart wurde.

Am Gerinnselfänger 29 kann durch die Folie 3 hindurch bzw. über die Folie 3 der Druck im extrakorporalen Kreislauf gemessen werden, also insbesondere nach Durchlaufen des Dialysators.

Die Kassette 1000 weist einen venösen Patientenanschluss 31 auf.
Die Kassette 1000 weist eine arterielle Heparin-Zugabestelle 33 auf. Dabei gilt zu beachten, dass die Heparin-Zugabestelle 33 (wie auch eine venöse Heparin-Zugabestelle 37) auch zur Zugabe anderer pharmakologischer Wirkstoffe als Heparin, welche nur vorzugsweise Antikoagulantien sind, oder Wirkstoffkombinationen geeignet und vorgesehen sein kann. Dies ist stets auch dann zu beachten, wenn zuvor oder im Folgenden von Heparin in beliebigem Zusammenhang die Rede ist.

Die Kassette 1000 weist ein Rückschlagventil 35 der arteriellen Heparin-Zugabestelle 33 auf.

Beispielhafte Rückschlagventile zum Einsatz als Rückschlagventil 35 der arteriellen Heparin-Zugabestelle 33 sowie als weitere Rückschlagventile der Kassette 1000 sind in der Patentanmeldung 10 2009 024 469.7 der Anmelderin der vorliegenden Erfindung mit dem Titel *"Ventilvorrichtung, Ventileinsatz*, *externe Funktionseinrichtung, Behandlarngsvorrichtung sowie Verfahren",* die am 10. Juni 2009 beim DPMA eingereicht wurde, offenbart.

Die Kassette 1000 weist ein arterielles Heparin-Zugabeventil 36 auf. Mit Hilfe des arteriellen Heparin-Zugabeventils 36 kann die Zugabe von Heparin in die arterielle Filterleitung 17 gesteuert oder geregelt werden.

Das arterielle Heparin-Zugabeventil 36 kann als so genanntes Phantomventil ausgestaltet sein.

Der Begriff "Phantomventil", wie er hierin verwendet wird, bezeichnet ein Element mit einer mittels Aktor erreichbaren Aktor-Fläche (hier beispielsweise eine Aktor-Membran), welches die Funktion eines Ventils übernehmen kann.

Die Aktor-Membran ist unter Aufbringung einer Kraft hierauf, z.B. einer Druckkraft, in eine Richtung bewegbar, ausdehnbar bzw. wölbbar oder dergleichen. Durch das Bewegen oder Ausdehnen der Aktor-Membran kann sich diese an ein Element, wie eine Abdichtungseinrichtung, etwa einen Steg, anlegen oder von diesem entfernen. Die Aktor-Membran kann somit beispielsweise eine Abdichtung bewirken bzw. verstärken oder beenden bzw. verringern.
Wenn die Kraft wieder von der Aktor-Membran genommen wird, kann diese in beispielsweise eine Grundposition, z.B. einen nicht gewölbten Zustand, zurückkehren.

Ein Phantomventil zur Verwendung als arterielles Heparin-Zugabeventil 36 sowie weitere Phantomventile der Kassette 1000 können mit oder aus einem Stegabschnitt eines Kanals am Hartteil 1 der Kassette 1000 und einem dem Stegabschnitt an- oder gegenüberliegenden Abschnitt der Folie 3 ausgestaltet werden.

Phantomventile können durch Aktoren der Blutbehandlungsvorrichtung betätigt werden.

Zum Schließen eines Phantomventils kann der Abschnitt der Folie 3 auf den Stegabschnitt gedrückt werden. Zum Öffnen des Phantomventils kann der Abschnitt der Folie 3 wieder vom Stegabschnitt abgehoben werden.

Weitere Beispiele und/oder Ausführungsformen für Phantomventile können der Patentanmeldung 10 2009 012 632.5 mit dem Titel *"Abdichtungseinrichtung zum Abdichten eines Volumens einer medizinischen Behandlungsanordnung gegen ein weiteres Volumen sowie Unordnung und Verfahren",* die am 10. März 2009 von der vorliegenden Anmelderin beim DPMA eingereicht wurde, entnommen werden.

Die Kassette 1000 weist eine venöse Heparin-Zugabestelle 37 auf. Die venöse Heparin-Zugabestelle 37 kann Luer-Konnektor ausgestaltet sein.

Die Kassette 1000 weist ein Rückschlagventil 39 der venösen Heparin-Zugabestelle 37 auf.

Die Kassette 1000 weist ein venöses Heparin-Zugabeventil 40 auf. Mit Hilfe des venösen Heparin-Zugabeventils 40 kann die Zugabe von Heparin in die venöse Filterleitung 19 gesteuert oder geregelt werden.

Die Kassette 1000 weist eine Substituat-Zugabestelle 41 bzw. einen Substituatkonnektor auf.

Die Substituat-Zugabestelle 41 kann eine Verbindungseinrichtung sein, wie in der Patentanmeldung 10 2009 024 575.8 der vorliegenden Anmelderin beschrieben ist, welche mit dem Titel *"Verbindungseinrichtung und Verfahren zumKonnektieren wenigstens zweier fluidführender medizintechnischer Systeme, sowie medizintechnische Vorrichtung"* am 10. Juni 2009 von der vorliegenden Anmelderin beim DPMA eingereicht wurde.

Die Substituat-Zugabestelle 41 kann mit einem Berührschutzelement (nicht gezeigt) versehen sein. Die Substituat-Zugabestelle 41 kann mit einem Tropfschutz (nicht gezeigt) versehen sein. Der Tropfschutz kann durch eine integrierte Verschlusshülse realisiert werden. Der Tropfschutz kann ein Heraustropfen von Resten von Substituat und/oder Blut beim Lösen der Kassette 1000 und anschließendem Entnehmen der Kassette 1000 aus der Blutbehandlungsvorrichtung verhindern.

Der Tropfschutz kann abnehmbar ausgeführt sein. Er kann als Haube oder Deckel ausgestaltet sein.

Die Substituat-Zugabestelle 41 oder ein anderer Abschnitt der Kassette 1000 kann ferner einen Originalitätsschutz bieten, durch welchen der Anwender ohne Mühe oder auf einen Blick erkennt, ob die Kassette 1000 bereits benutzt wurde. Dieser Originalitätsschutz kann mittels des Berührschutzelements, der Verschlusshülse oder einer anderen Struktur realisiert sein. Die entsprechende Struktur kann vorzugsweise ihre Position innerhalb der oder bezogen auf die Kassette 1000 erkennbar verändern. Sie kann vorzugsweise ihre Gestalt verändern.

Zudem kann die Substituat-Zugabestelle 41 oder ein anderer Abschnitt der Kassette 1000 einen Wiederverwendungsschutz bzw. Schutz gegen ein Wiederverwenden bieten. Vorzugsweise durch eine Verschlusshülse wird die Kassette 1000 - vorzugsweise irreversibel - im Hinblick auf den Versuch einer Wiederverwendung unbrauchbar gemacht. Soll die Kassette 1000 trotzdem erneut verwendet werden wollen, so messen Sensoren der Blutbehandlungsvorrichtung nicht die Signalverläufe, die bei Verwenden einer neuen Kassette gemessen werden würden. Dies kann hierauf beruhen, dass keine Flüssigkeit in die Kassette 1000 oder in die Substituat-Zugabestelle 41 gelangen kann oder wenigstens nicht in ausreichender oder üblicher Menge. Die Steuereinheit der Blutbehandlungsvorrichtung kann dies erkennen. Es kann eine Warnung veranlasst werden.

Als Originalitätsschutz oder Wiederverwendungsschutz kann vorzugsweise ein Originalitätsschutz oder ein Wiederverwendungsschutz verwendet werden, wie er in der von der Anmelderin der vorliegenden Erfindung in der Patentanmeldung 10 2009 024 575.8 mit dem Titel *"Verbindungseinrichtung und Verfahren zumKonnektieren wenigstens zweier fluidführender medizintechnischer Systeme, sowie medizintechnische Vorrichtung",* die am 10. Juni 2009 beim DPMA eingereicht wurde, offenbart wurde.

Die Kassette weist einen Konnektor 43 für einen Substituataustritt aus der Kassette 1000 sowie einen Konnektor 45 für einen Sustituateintritt in die Kassette 1000 auf.

Die Konnektoren 43 und 45 sind mit einem Pumpschlauchsegment oder -set einer Substituatpumpe verbindbar.

Die Kassette 1000 weist ein Rückschlagventil 47 für Substituatzugabe auf.

Über Betätigung des Rückschlagventils 47 kann Substituat in eine Substituatleitung 49 eingebracht werden.

Die Kassette 1000 weist ein Prädilutions-Zugabeventil 51 auf. Das Prädilutions-Zugabeventil 51 kann als Phantomventil ausgestaltet sein.

Die Kassette 1000 weist ein Postdilutions-Zugabeventil 53 auf. Das Postdilutions-Zugabeventil 53 kann als Phantomventil ausgestaltet sein.

Die Kassette 1000 weist eine Single-Needle-Sterilmembran 55 auf.

Die Kassette 1000 weist eine Single-Needle-Kammer 57 auf. Die Single-Needle-Kammer 57 ist in Fig. 1 oberhalb der venösen Blutkammer 21 angeordnet.

Im Inneren der Single-Needle-Kamer 57 ist ein Blutschwallumleitungselement 59 angeordnet. Das Blutschwallumleitungselement 59 kann dem Abbremsen eines Blutschwalls und/oder dem Auslöschen dessen Impulses dienen.

Eine Verbindung mit einem Inneren der Single-Needle-Kammer 57 kann mittels einer Verbindungseinrichtung vorgesehen sein, wie sie in der von der Anmelderin der vorliegenden Erfindung mit dem Titel *"Einrichtung sowie externe Funktionseinrichtung und Behandlungsvorrichtung zumBehandeln von medizinischen Fluiden"* 10 2009 024 467.0 am 10. Juni 2009 beim DPMA eingereichten Patentanmeldung offenbart wurde.

Die Kassette 1000 weist ein Single-Needle-Blutventil 61 auf. Das Single-Needle-Blutventil 61 kann als Phantomventil ausgestaltet sein.

Die Kassette 1000 weist eine Absaugstelle 63 auf. Die Absaugstelle 63 kann zur Vakuumankopplung der Kassette 1000 an die Blutbehandlungsvorrichtung dienen, wie dies beispielsweise in der Patentanmeldung DE 10 2007 042 964 A1 mit dem Titel *vorrichtung und Verfahren zur Behandlung einer medizinischen Flüssigkeit",* die am 10. September 2007 beim DPMA eingereicht wurde, beschrieben ist.

Die Kassette 1000 weist ein primäres Ausrichtungszentrum 65 auf. Das primäre Ausrichtungszentrum 65 kann vorteilhaft zum Ausrichten und/oder Verrasten der Kassette 1000 an der Blutbehandlungsvorrichtung dienen.

Die Kassette 1000 weist eine sekundäre Ausrichtungsstelle 67 auf. Die sekundäre Ausrichtungsstelle 67 kann zum Ausrichten und/oder Verrasten der Kassette 1000 an der Blutbehandlungsvorrichtung dienen.

Die Kassette 1000 ist vor Beginn des Primens mit Gas (z.B. steriler Luft) gefüllt. Beim Primen des extrakorporalen Blutkreislaufs muss diese Gasfüllung verdrängt werden. Eine Blutbehandlungskassette stellt hierbei allgemein eine besondere Herausforderung dar, weil es sowohl aufsteigende als auch fallende Leitungen und zudem Kammern gibt, in denen keine "Luftnester" verbleiben dürfen. Die vorliegende Kassette 1000 weist zu diesem Zweck besondere Konstruktionsmerkmale auf:

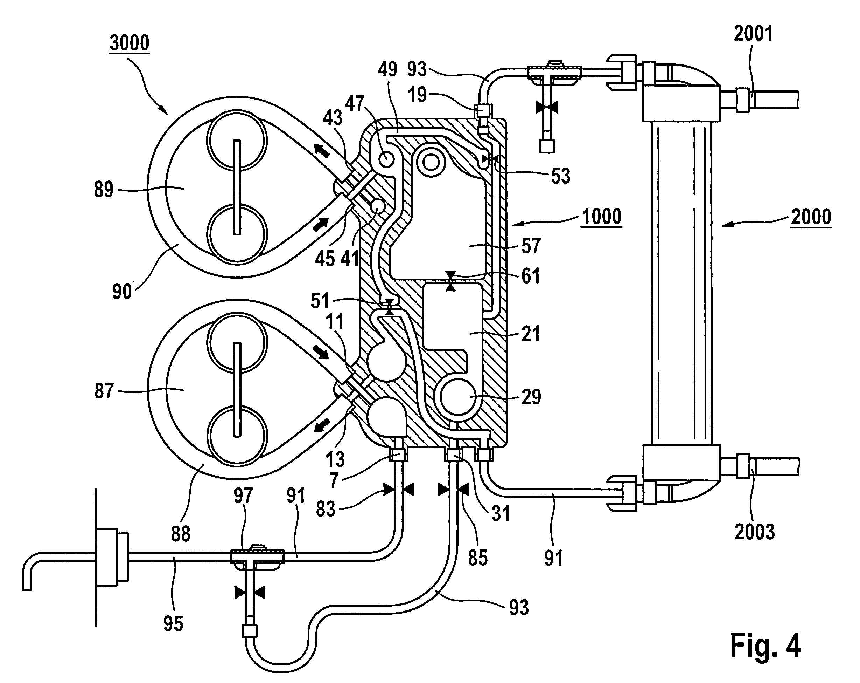

Die Kammer 15 zum Messen des arteriellen Drucks ist derart konstruiert, dass die gesamte Luft in ein Pumpschlauchsegment (z.B. in das Pumpschlauchsegment 90, siehe z.B. Fig. 4) aufsteigen kann. Es gibt hierbei vorteilhaft keine Toträume. Luft, die aus der arteriellen Druckmesskammer in das Pumpschlauchsegment der Blutpumpe selbständig aufsteigt, wird ab dem Eingriffsbereich Blutpumpe (z.B. durch die Rollen einer Rollenpumpe) durch das Pumpschlauchsegment zwangsgefördert. Sobald die Pumpe keinen Einfluss mehr hat (weil z.B. die Rollen in Ausgriff gehen) steigt die Luft selbsttätig in Förderrichtung in die Kassette 1000 auf.

Die venöse Rückführleitung (bzw. ein venöser Abschnitt 93 des extrakorporalen Kreislaufs wie u.a. in Fig. 4 gezeigt) ist eine Fallleitung. Ab einem gewissen Volumenstrom (z.B. 200 ml/min bei der in Fig. 1 gezeigten Kassette 1000) werden Luftblasen im Blut auch entgegen der Erdbeschleunigung "mitgerissen". Dieser Effekt wird bei den Fallleitungen ausgenutzt. Die Leitungsquerschnitte der Fallleitungen sind so klein ausgelegt, dass durch die Strömungsgeschwindigkeit eine Zwangsförderung der Luftblasen auch entgegen der Erdbeschleunigung funktioniert.

In der venösen Blutkammer 21 sind große Querschnitte vorgesehen, derart, dass aufgrund der dort langsamen bzw. niedrigen Strömungsgeschwindigkeiten Luftblasen verlässlich entgegen der Hauptströmungsrichtung aufsteigen können.

Weitere konstruktive Besonderheiten der Kassette 1000 sind:
Die Phantomventile 40, 51 und 53 sind räumlich derart ausgerichtet, dass Blut (welches eine höhere Dichte als Wasser bzw. Substituat etc. hat) bei Betrieb der Kassette 1000 mit Blut kaum "nach oben" oder "zur Seite" in geöffnete Phantomventile eindringen kann, weil es gegenüber dem leichteren Wasser absinkt. Eine solch vorteilhafte Ausrichtung ist durch die Phantomventile 40, 51, und 53 realisiert. Beim Ventil 36 besteht hingegen kein solches Erfordernis, d.h. dort kommt es auf die Orientierung nicht an.

Der Leitungskanal (Stichkanal) unter dem Rückschlagventil 47 für Substituatzugabe ist aus demselben Grunde aufsteigend konstruiert. Im Falle einer Fehlfunktion der Prä- und/oder Postdilutionsventile 51 und 53 und einer daraus resultierenden Blut-Bypassströmung kann kein Blut in die Substituatleitung 49 aufsteigen. Vielmehr strömt das Blut an der Mündung der entsprechenden Stichleitung vorbei.

Die Neigung der Kassette 1000 beträgt vorzugsweise 5° bis 11°, besonders bevorzugt die bereits genannten 8°.

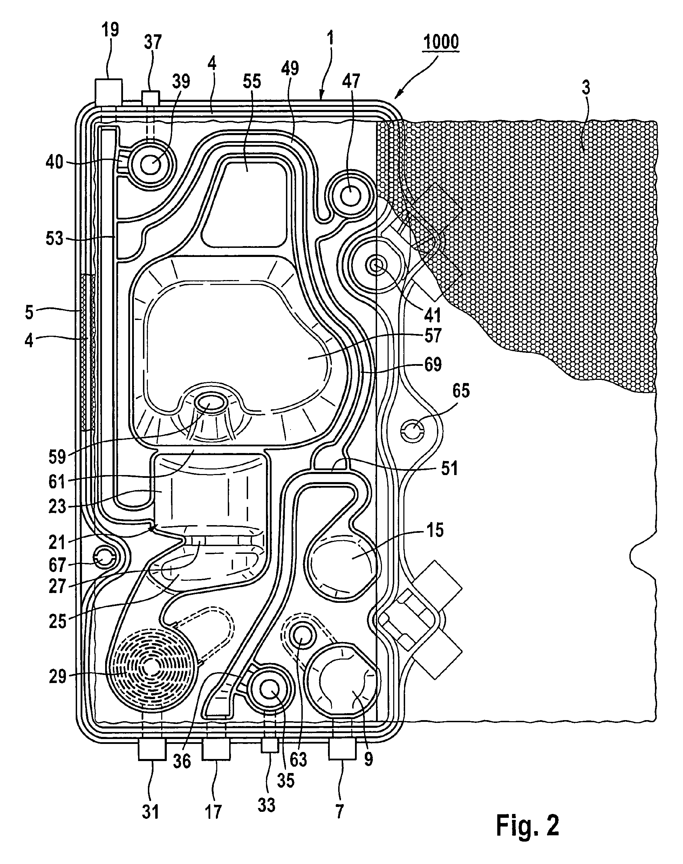

Fig. 2 zeigt die Kassette 1000 der Fig. 1, wobei die Folie 3 am linken Rand der Kassette 1000 sowie oben und unten zur besseren Illustration zerstörend aufgeschnitten und nach rechts aufgeklappt zu erkennen ist.

Wie in Fig. 2 gezeigt, weist die Folie 3 eine Oberflächenstrukturierung auf.

Fig. 2 zeigt die nach Aufschneiden der Folie 3 detaillierter zu erkennenden Elemente im Inneren der Kassette 1000.

Zur Vermeidung von Wiederholungen wird auf die vorstehend bei der Beschreibung der Fig. 1 erörterten Ausgestaltungen der einzelnen Elemente verwiesen.

Gut zu erkennen ist hierbei, dass die Kassette 1000 einen Dichtsteg 69 aufweist. Der Dichtsteg 69 kann zum Beispiel zum Ausgestalten des Prädilutions-Zugabeventils 51 eingesetzt werden.

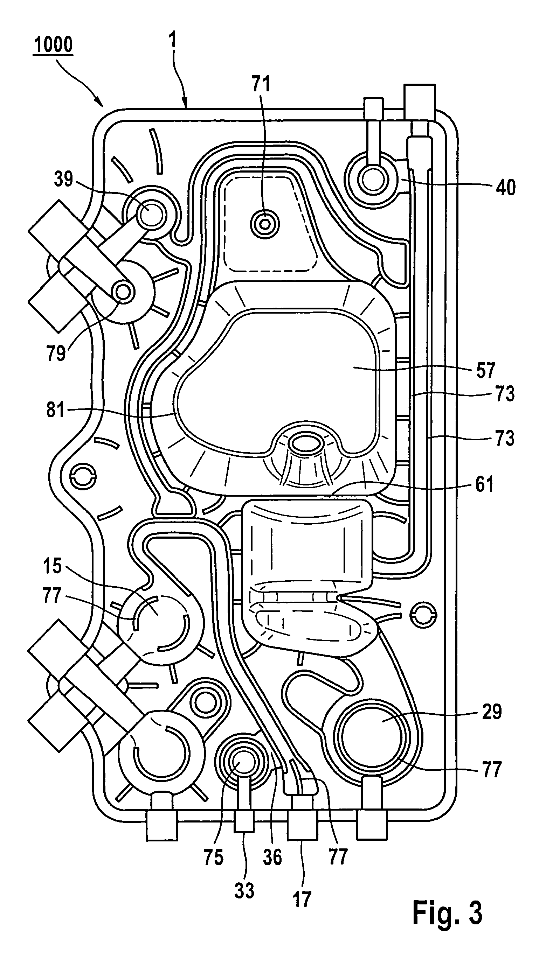

**Fig. 3** zeigt die Kassette 1000 von ihrer Rückseite. Ist die Kassette 1000 an die Blutbehandlungsvorrichtung angekoppelt, so wird ein Betrachter beim Öffnen einer Tür der Blutbehandlungsvorrichtung zum Entnehmen der Kassette 1000 auf diese Rückseite blicken.

Die Kassette 1000 weist einen Single-Needle-Luft-Konnektor 71 auf. Es kann vorgesehen sein, geräteseitig und/oder blutseitig ein Abstützgitter (nicht gezeigt) der Single-Needle-Sterilmembran 55 am Single-Needle-Luft-Konnektor 71 anzuordnen.

Die Kassette 1000 weist mehrere Stützstege auf. Die Stützstege weisen von einander verschiedene Höhen, bezogen beispielsweise auf die Ebene der Folie 3, auf. Die Stützstege erheben sich in der dem Betrachter in Fig. 3 zugewandten Seite der Kassette 1000, also aus der Zeichenebene der Fig. 3 heraus.

Die Kassette 1000 weist Stützstege 73 mit einer Höhe von 5 mm, Stützstege 75 mit einer Höhe von 8 mm, Stützstege 77 mit einer Höhe von 13 mm, Stützstege 79 mit einer Höhe von 24 mm und Stützstege 81 mit einer Höhe von 31 mm auf. Die vorstehenden und andere Zahlenwerte sind natürlich rein exemplarisch zu verstehen.

Die Stützstege können dazu dienen, die Kassette im angekoppelten Zustand an eine Blutbehandlungsvorrichtung gegen einen Deckel einer Aufnahmeeinrichtung der Blutbehandlungsvorrichtung zum Aufnehmen der Kassette abzustützen. Beispielhafte Ausführungsformen einer derartigen Ankopplung der Kassette an die Blutbehandlungsvorrichtung sind in der Patentanmeldung 10 2009 012 633.3 mit dem Titel *"Vorrichtung zum Verbinden einer* externen *Funktionseinrichtung mit einer Anordnung, Anordnung aufweisend eine solche Vorrichtung und Verfahren zum Verbinden",* die am 10. März 2009 beim DPMA eingereicht wurde, angegeben.

In Fig. 3 ist die Kassette 1000 so gezeigt, wie der Anwender/Betrachter nach ihrem Ankoppeln an das Maschineninterface auf sie blickt. Die Neigung der Kassette 1000 zur Maschine ist "nach hinten geneigt" ausgeführt, so dass die obere Kante weiter vom Anwender/Betrachter entfernt ist als die untere Kante.

Die nach oben zeigenden Flächen der venösen Blutkammer 21 und der Single-Needle-Kammer 57 weisen dementsprechend eine Neigung derart auf, dass Luftblasen trotz der Neigung der Kassette 1000 noch zuverlässig an der Innenseite aufsteigen können. Grundsätzlich ist alternativ natürlich auch ein Kassettendesign möglich, das keine Neigung der Kassette vorsieht.

Im Folgenden werden anhand der **Fig. 4 bis 16** verschiedene Prozesse beschrieben, welche mittels der erfindungsgemäßen externen *Funktionseinrichtung* bei Blutbehandlungsverfahren einsetzbar sind.

Die Blutbehandlungsvorrichtung weist eine externe Funktionseinrichtung, zum Beispiel die in den Figuren 1 bis 3 erläuterte Kassette 1000 mit den vorstehend in den Figuren 1 bis 3 beschriebenen Elementen, auf.

Die Blutbehandlungsvorrichtung weist ferner eine Dialysiereinrichtung 2000 mit einem Dialysierflüssigkeitseintritt 2001 und einem Dialysierflüssigkeitsaustritt 2003 auf.

Die Blutbehandlungsvorrichtung weist zudem einen extrakorporalen Kreislauf 3000 auf. Der extrakorporale Kreislauf 3000 weist eine arterielle Patientenschlauchklemme 83 und eine venöse Patientenschlauchklemme 85 auf.

Der extrakorporale Kreislauf 3000 weist eine Blutpumpe 87 mit einem Pumpschlauchsegment 88 auf.

Der extrakorporale Kreislauf 3000 weist eine Substituatpumpe 89 mit einem Pumpschlauchsegment 90 auf.

Die Blutpumpe 87 und die Substituatpumpe 89 können als peristaltische Pumpen, beispielsweise Rollenpumpen wie in den Figuren, ausgestaltet sein.

Der Begriff "Förderrichtung" bzw. "Strömungsrichtung" bezeichnet die während einer Blutbehandlung übliche Förderrichtung des zu reinigenden Bluts von einem Patienten zu einer Dialysiereinrichtung und des gereinigten Bluts von der Dialysiereinrichtung in den Patienten zurück. Diese Förderrichtung verläuft in der Zeichenebene der Figur gegen den Uhrzeigersinn.

Analog kennzeichnen die Begriffe "Förderrichtung" oder "Strömungsrichtung" in Zusammenhang mit der Strömung eines Substituats eine während einer Blutbehandlung übliche Förderrichtung des Substituat aus dem Substituat-Zugabeventil 41 in den extrakorporalen Kreislauf 3000.

Eine gegen diese Förderrichtung erfolgende Fluidförderung (insbesondere Blut und Substituat) wird als Förderung bzw. Strömung in Gegenrichtung bezeichnet.

Der extrakorporale Kreislauf 3000 weist einen arteriellen Abschnitt 91 und einen venösen Abschnitt 93 auf.

Der arterielle Abschnitt 91 des extrakorporalen Kreislaufs 3000 erstreckt sich von einem Abschnitt zum arteriellen Konnektieren eines Patienten, zum Beispiel einer arteriellen Nadel, durch die Kassette 1000 bis zu einem Bluteintritt an der Dialysiereinrichtung 2000. Der arterielle Abschnitt 91 weist verschiedene Komponenten auf. So bilden eine arterielle Konnektion eines Patienten, der arterielle Patientenanschluss 7, die arterielle Patientenschlauchklemme 83, die arterielle Druckmesskammer 9, die Kammer 15 mit arterieller Post- bzw. Präfilter-Druckmessstelle, das Pumpschlauchsegment 88 der Blutpumpe 87, die arterielle Filterleitung 17 und ein Bluteintritt an der Dialysiereinrichtung 2000 jeweils Teil des arteriellen Abschnitts 91 des extrakorporalen Kreislaufs 3000.

Der venöse Abschnitt 93 des extrakorporalen Kreislaufs 3000 erstreckt sich von einem Blutaustritt an der Dialysiereinrichtung 2000 bis zu einem Abschnitt zum venösen Konnektieren eines Patienten, zum Beispiel einer venösen Nadel. Der venöse Abschnitt 93 weist verschiedene Komponenten auf. So bilden ein Blutaustritt aus der Dialysiereinrichtung 2000, die venöse Filterleitung 19, die venöse Blutkammer 21, der Gerinnselfänger 29, die Single-Needle-Kammer 57, der venöse Patientenanschluss 31, die venöse Patientenschlauchklemme 85 und eine venöse Konnektion eines Patienten jeweils Teil des venösen Abschnitts 93 des extrakorporalen Kreislaufs 3000.

**Fig. 4** zeigt eine Phase während der Durchführung eines Vorbereitungsprozesses bzw. Priming-Prozesses zum Befüllen der eingesetzten Fluidleitungen gemäß einem hierin beschriebenen Verfahren.

Der arterielle Abschnitt 91 und der venöse Abschnitt 93 des extrakorporalen Kreislaufs 3000 sind miteinander verbunden.

Das Prädilutions-Zugabeventil 51, das Postdilutions-Zugabeventil 53 und das Single-Needle-Blutventil 61 der Kassette 1000 sind geöffnet. Die beiden Patientenschlauchklemmen 83 und 85 sind ebenfalls geöffnet.

Fig. 4 zeigt die beschriebene Konfiguration als Momentaufnahme bzw. eine Phase während des Vorbereitungs- oder Priming-Prozesses.

Über die Substituat-Zugabestelle 41 wird Substituat in den extrakorporalen Kreislauf 3000 eingebracht. Dazu wird der automatische Substituat-Konnektor konnektiert. Der arterielle Patientenschlauch im arteriellen Abschnitt 91 des extrakorporalen Kreislaufs 3000 und der venöse Patientenschlauch im venösen Abschnitt 93 des extrakorporalen Kreislaufs 3000 werden mit einem Rinse-Port der Blutbehandlungsvorrichtung verbunden, z. B. mittels eines geeigneten Verbinders, mit dem für das Ende des einen Patientenschlauchs ein Zugang in den anderen Patientenschlauch geschaffen wird. Das Ende des anderen Patientenschlauchs dient als Ablaufleitung in den Rinse-Port. Der Verbinder kann sich alternativ in der arteriellen oder der venösen Patientenleitung, d.h. dem arteriellen Abschnitt 91 oder dem venösen Abschnitt 93, befinden.

Die venöse Patientenschlauchklemme 85 wird geschlossen, das Postdilutions-Zugabeventil 53 wird geöffnet, das Prädilutions-Zugabeventil 51 wird geschlossen.

Das Füllen der venösen Blutkammer 21 erfolgt mittels der Substituatpumpe 89 durch das Postdilutions-Zugabeventil 53. Dabei erfolgt eine Luftabscheidung durch das Single-Needle-Blutventil 61.

Die Blutpumpe 87 läuft vorwärts und saugt aus der venösen Blutkammer 21 Substituat an.

Wenn der Pegel in der venösen Blutkammer 21 sinkt, so wird über das Postdilutions-Zugabeventil 53 nachgefördert, bis ein Überschreiten der vorgegebenen Füllhöhe vom Level-Detektor erkannt wird. Während dieses sich bei Bedarf wiederholenden Vorgangs läuft die Blutpumpe 87 kontinuierlich weiter.

Der Gerinnselfänger 29 wird " von unten" entlüftet: Alle drei Kassettenventile (51, 53, 61) sind geschlossen. Die arterielle Patientenschlauchklemme 83 ist geöffnet und die venöse Patientenschlauchklemme 85 ist geschlossen. Der Rinse-Port ist geschlossen.
Die Blutpumpe 87 läuft kurz rückwärts und fördert ein kleines Volumen. Dadurch wird venös ein Unterdruck und arteriell ein Überdruck im extrakorporalen Kreislauf 3000 erzeugt.

Die venöse Patientenschlauchklemme 85 wird solange geöffnet, bis ein Druckausgleich stattgefunden hat.

Anschließend wird das Füllen des extrakorporalen Kreislaufs 3000 fortgesetzt.

Mittels des Sensors/Detektors 115 an der venösen Patientenschlauchklemme 85, z.B. eines venösen Luftblasendetektors, wird das Auftreten von Luftblasen detektiert. Sobald im Verlauf eines vorgegebenen Zeitintervalls keine Luftblasen oder nahezu keine Luftblasen detektiert wurden, gilt der extrakorporale Kreislauf 3000 als gefüllt.

Dann wird der gefüllte extrakorporalen Kreislauf 3000 gespült. Während des Spülens wird Substituat durch das Prädilutions-Zugabeventil 51 gefördert und durch den Rinse-Port ("Hahn 97") verworfen.

Dabei sind sowohl die arterielle Patientenschlauchklemme 83 als auch die venöse Patientenschlauchklemme 85 geöffnet. Die Blutpumpe 87 läuft rückwärts und fördert einen Teil des Substituats in den Rinse-Port.

Alternativ zum Online- Füllen (das Substituat wird dabei online in der Dialysemaschine bereitgestellt) ist, wie vorstehend bereits angegeben, auch das Füllen mit einem externen Beutel mit Kochsalzlösung als Quelle für die Befüllflüssigkeit möglich. Dazu wird die arterielle Patientenleitung bzw. der arterielle Abschnitt 91 des extrakorporalen Kreislaufs 3000 an den Beutel mit Kochsalzlösung angeschlossen. Die venöse Patientenleitung bzw. der venöse Abschnitt 93 des extrakorporalen Kreislaufs 3000 wird an einem sog. Waste-Bag als Senke für die gebrauchte Kochsalzlösung angeschlossen. Die Blutpumpe 87 läuft vorwärts. Durch das Öffnen des Prädilutions-Zugabeventils 51 und des Postdilutions-Zugabeventils 53 kann auch die zwischen diesen beiden Ventilen befindliche Leitung befüllt werden.

Bei beiden Verfahren wird der Patient erst nach Erreichen einer vorgegebenen Spülmenge an den extrakorporalen Kreislauf 3000 angeschlossen.

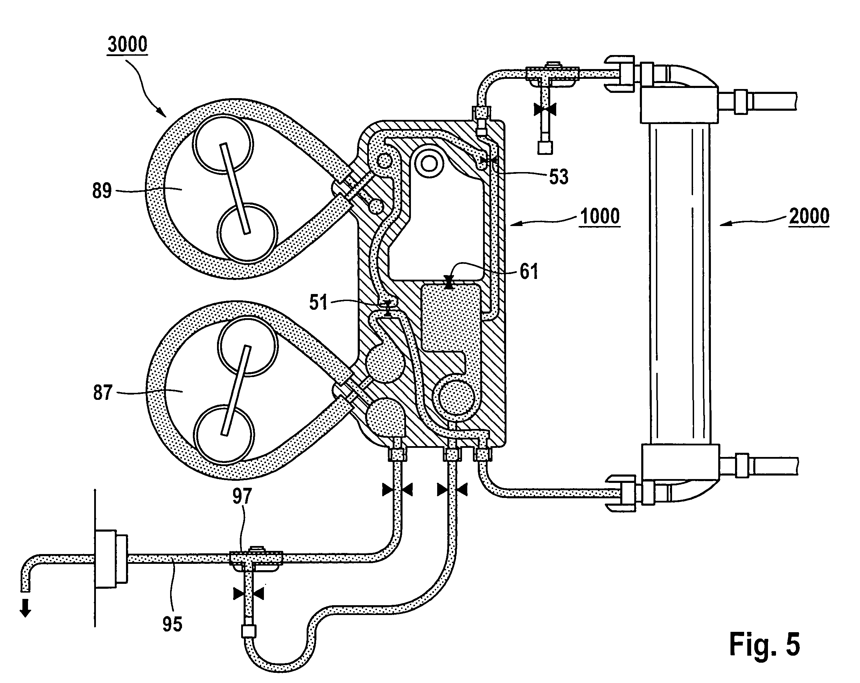

**Fig. 5** zeigt eine Phase eines Spülungsprozesses bzw. Rinsing-Prozesses gemäß einem hierin beschriebenen Verfahren.

Um das nach der Vorbereitung bzw. dem Befüllen (Priming) im nunmehr geschlossenen extrakorporalen Kreislauf 3000 zirkulierende Substituat aus diesem zu entfernen, wird der Hahn 97 geöffnet.

Die Substituatpumpe 89 wird erneut eingeschaltet. Das Prädilutions-Zugabeventil 51 wird geöffnet.

Die Blutpumpe 87 und die Substituatpumpe 89 fördern das Substituat über die Ablaufleitung 95 aus dem extrakorporalen Kreislauf 3000 heraus.

Fig. 5 zeigt die beschriebene Konfiguration als Momentaufnahme bzw. in einer Phase des Spülungs- oder Rinsing-Prozesses.

Die Blutpumpe 87 und die Substituatpumpe 89 fördern ständig neues Substituat, so dass der extrakorporale Kreislauf 3000 gespült wird. Das gebrauchte Substituat wird verworfen.

Die Blutpumpe 87 und die Substituatpumpe 89 fördern jeweils im Uhrzeigersinn. Die Blutpumpe 87 und die Substituatpumpe 89 können gegeneinander versetzt fördern. Die Substituatpumpe 89 kann schneller als die Blutpumpe 87 rotieren.

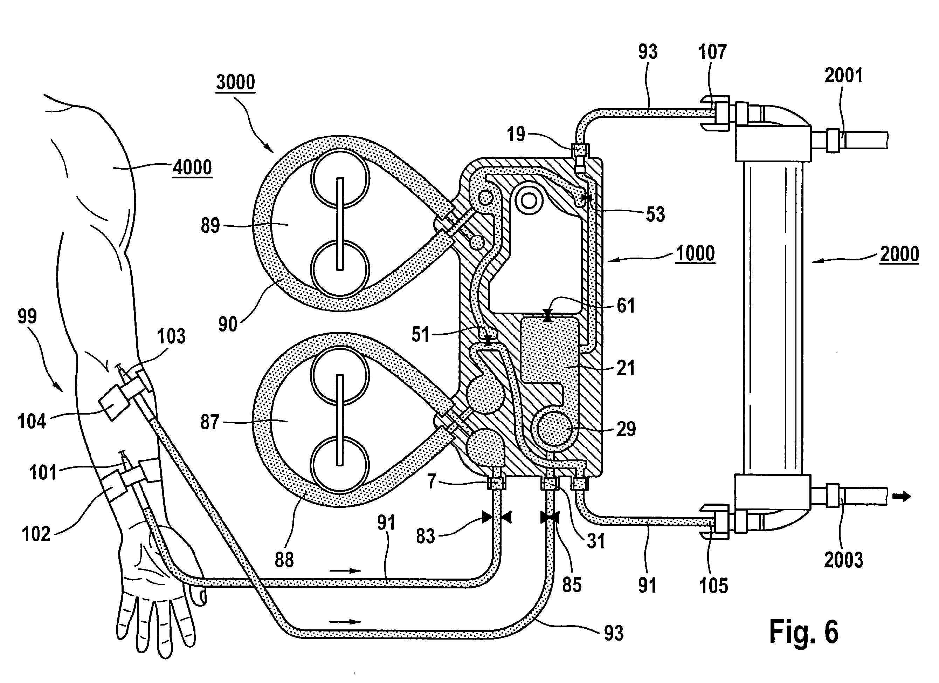

**Fig. 6** zeigt eine Phase beim Anschließen eines Patienten 4000 an den extrakorporalen Kreislauf 3000 gemäß einem hierin beschriebenen Verfahren auf eine erste Weise mittels eines Double-Needle-Zugangs 99. Fig. 6 stellt daher wiederum - wie alle Figuren zu Verfahrensschritten - nur eine Phase dar.

Zum Anschließen eines Patienten 4000 an die Blutbehandlungsvorrichtung wird ein Double-Needle-Zugang 99 verwendet.

Der Double-Needle-Zugang 99 weist eine arterielle Nadel 101 mit einer Fixierung 102, z.B. einer Manschette, einem Pflaster und dergleichen, und eine venösen Nadel 103 mit einer Fixierung 104, z.B. eine Manschette, ein Pflaster und dergleichen, auf.

Die arterielle Nadel 101 ist mit dem arteriellen Patientenanschluss 7 der Kassette 1000 verbunden. Die venöse Nadel 103 ist mit dem venösen Patientenanschluss 31 der Kassette verbunden.

Die venöse Nadel 103 wird dem Patienten 4000 gelegt und fixiert. Die arterielle Nadel 101 wird dem Patienten 4000 gelegt und fixiert. Die venöse Nadel 103 kann vor der arteriellen Nadel 101 mit dem Patienten 4000 konnektiert werden.

Der extrakorporale Kreislauf 3000 aufgrund seiner Befüllung ist mit Substituat gefüllt. Das Prädilutions-Zugabeventil 51, das Postdilutions-Zugabeventil 53 und das Single-Needle-Blutventil 61 sind geschlossen.

Zunächst sind beide Patientenschlauchklemmen 83 und 85 geschlossen.

Die Blutpumpe 87 wird betätigt. Die arterielle Patientenschlauchklemme 83 wird geöffnet.

Fig. 6 zeigt die beschriebene Konfiguration als Momentaufnahme mit bereits geöffneter arterieller Patientenschlauchklemme 83, kurz vor einem Starten der Blutpumpe 87.

Vom Patienten 4000 wird Blut über die arterielle Nadel 101 in den arteriellen Abschnitt 91 des extrakorporalen Kreislaufs 3000 gefördert und verdrängt oder fördert das Substituat. Das Substituat wird über den Dialysierflüssigkeitsaustritt 2003 der Dialysiereinrichtung 2000 aus der Blutbehandlungsvorrichtung gefördert.

Wenn das Blut aus dem arteriellen Abschnitt 91 des extrakorporalen Kreislaufs 3000 an einem Bluteintritt 105 an der Dialysiereinrichtung 2000 ankommt, wird die arterielle Patientenschlauchklemme 83 geschlossen und die Blutpumpe 87 gestoppt.

Die venöse Patientenschlauchklemme 85 wird geöffnet.

Über die venöse Nadel 103 tritt Blut in Gegenrichtung in den venösen Abschnitt 93 des extrakorporalen Kreislaufs 3000 ein. Die Blutpumpe 87 wird nicht betätigt.

Das Blut kann beispielsweise bedingt durch die Schwerkraft in den venösen Abschnitt 93 eintreten.

Das Blut durchströmt den venösen Abschnitt 93 in Gegenrichtung und gelangt in den Gerinnselfänger 29 und die venöse Blutkammer 21. Das Blut durchströmt die venöse Filterleitung 19 und gelangt in Gegenrichtung durch den Blutaustritt 107 in die Dialysiereinrichtung 2000.

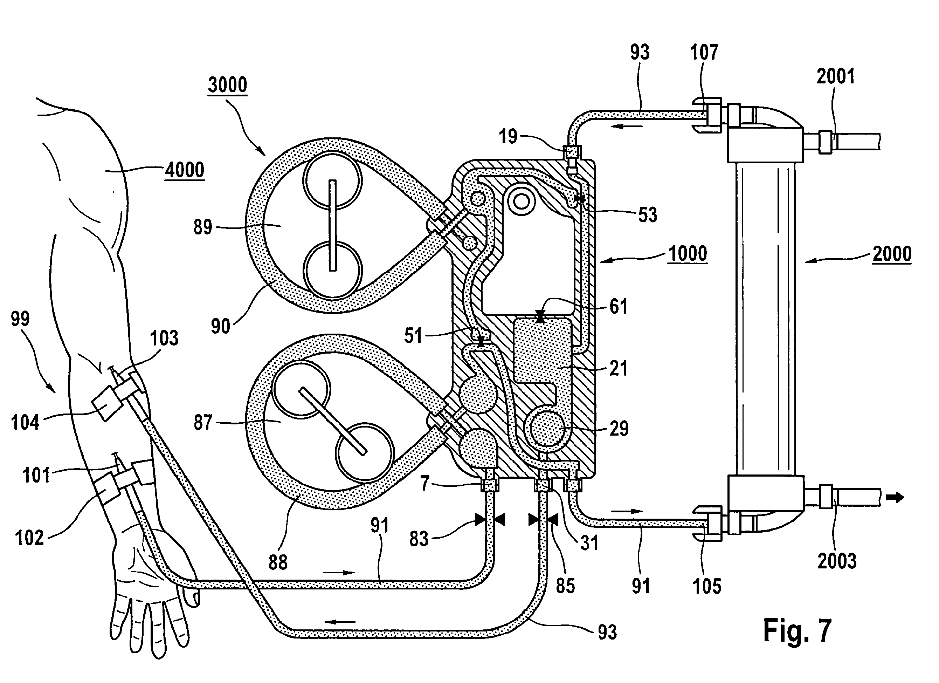

**Fig. 7** veranschaulicht eine Phase einer zweiten Weise bzw. eines alternativen Prozesses zum Anschließen eines Patienten 4000 an eine Blutbehandlungsvorrichtung.

Der Patient 4000 ist über eine arterielle Nadel 101 und eine venöse Nadel 103 mit dem extrakorporalen Kreislauf 3000 konnektiert.

Die arterielle Patientenschlauchklemme 83 und die venöse Patientenschlauchklemme 85 sind geöffnet. Das Prädilutions-Zugabeventil 51, das Postdilutions-Zugabeventil 53 und das Single-Needle-Blutventil 61 sind geschlossen.

Im gesamten extrakorporalen Kreislauf 3000 befindet sich Substituat (außer in der Single-Needle-Kammer 57).

Die Blutpumpe 87 wird gestartet. Die Dialysiereinrichtung 2000 wird nicht eingeschaltet.

Fig. 7 zeigt die beschriebene Konfiguration als Momentaufnahme. Es befindet sich zu diesem Zeitpunkt ausschließlich Substituat im extrakorporalen Kreislauf 3000.

Aus dem Patienten 4000 wird unter Betreiben der Blutpumpe 87 Blut durch die arterielle Nadel 101 in den arteriellen Abschnitt 91 des extrakorporalen Kreislaufs 3000 gefördert. Das Blut strömt am Bluteintritt 105 an der Dialysiereinrichtung 2000 in diese ein und von dort durch den Blutaustritt 107 der Dialysiereinrichtung 2000 in den venösen Abschnitt 93 des extrakorporalen Kreislaufs 3000.

Über die venöse Filterleitung 19 gelangt das Blut in die Kassette 1000 und tritt bei geschlossenem Postdilutions-Zugabeventil 53 in die venöse Blutkammer 21 ein, wobei das Blut tangential in den oberen Raum der venösen Blutkammer 21 einströmen kann.

Aus der venösen Blutkammer 21 gelangt das Blut über den Gerinnselfänger 29 aus der Kassette 1000 über den venösen Patientenanschluss 103 zum Patienten 4000 zurück.

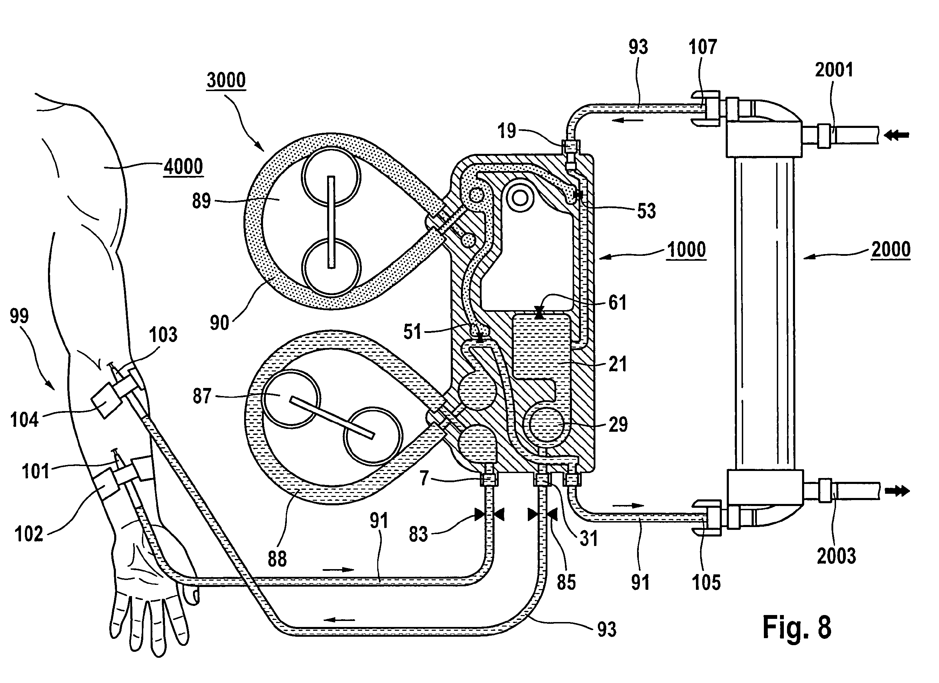

**Fig. 8** zeigt eine Phase einer Dialysebehandlung unter Verwenden eines Double-Needle-Zugangs zum Patienten.

Die arterielle Patientenschlauchklemme 83 und die venöse Patientenschlauchklemme 85 sind geöffnet. Das Prädilutions-Zugabeventil 51, das Postdilutions-Zugabeventil 53 und das Single-Needle-Blutventil 61 sind geschlossen.

Fig. 8 zeigt die beschriebene Konfiguration als Momentaufnahme während einer Dialysebehandlung unter Betreiben der Blutpumpe 87.

Die Dialysiereinrichtung 2000 wird betrieben, so dass Dialysierflüssigkeit am Dialysierflüssigkeitseintritt 2001 in die Dialysiereinrichtung 2000 eintritt. In der Dialysiereinrichtung erfolgt die Behandlung des Patientenblutes. Die Dialysierflüssigkeit tritt am Dialysierflüssigkeitsaustritt 2003 aus der Dialysiereinrichtung 2000 aus. Verbrauchte Dialysierflüssigkeit kann verworfen oder aufgereinigt werden.

Die Blutpumpe 87 fördert Blut vom Patienten 4000 über die arterielle Nadel 101 in den arteriellen Abschnitt 91 des extrakorporalen Kreislaufs 3000, wobei das Blut die Kassette 1000 durchströmt und zu der Dialysiereinrichtung 2000 gefördert wird.

Das Blut durchströmt die Dialysiereinrichtung 2000 in Gegenrichtung zur Dialysierflüssigkeit und wird dabei gereinigt. Am Blutaustritt 107 der Dialysiereinrichtung 2000 verlässt das gereinigte Blut die Dialysiereinrichtung 2000 und strömt durch die venöse Filterleitung 19 in die Kassette 1000, gelangt in die venöse Blutkammer 21 und den Gerinnselfänger 29 und tritt am venösen Patientenanschluss 31 aus der Kassette 1000 aus.

Über den venösen Patientenanschluss 103 wird das gereinigte Blut wieder in den Patienten 4000 rückgeführt.

Es erfolgt keine Zufuhr von Substituat. Die Substituatpumpe 89 wird nicht betrieben.

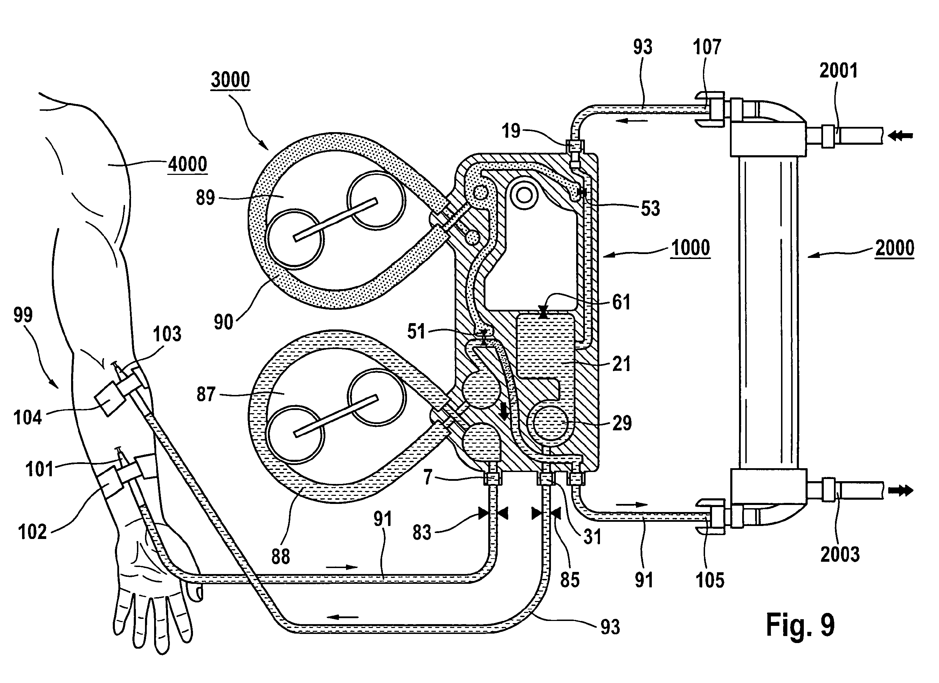

**Fig. 9** zeigt eine Phase des anhand von Fig. 8 dargestellten Blutbehandlungsprozess unter Verwenden einer Prädilution des Bluts mit Substituat ("Online HDF Prädilution").

Das Prädilutions-Zugabeventil 51 wird geöffnet. Das Postdilutions-Zugabeventil 53 und das Single-Needle-Blutventil 61 sind geschlossen. Die Blutpumpe 87 wird betrieben. Die Substituatpumpe 89 wird gestartet. Die Substituatpumpe 89 kann in Gleichtakt mit der Blutpumpe 87 betrieben werden.

Die beschriebene Konfiguration ist als Momentaufnahme in Fig. 9 gezeigt, wobei sich zwischen Prädilutions-Zugabeventil 51 und Postdilutions-Zugabeventil 53 Substituat befindet und der restliche extrakorporale Kreislauf 3000 von Blut durchströmt wird.

Die Substituatpumpe 89 fördert Substituat, welches am Prädilutions-Zugabeventil 51 in den arteriellen Abschnitt 91 des extrakorporalen Kreislaufs 3000 gelangt und sich mit dem zu reinigenden Blut vermischt.

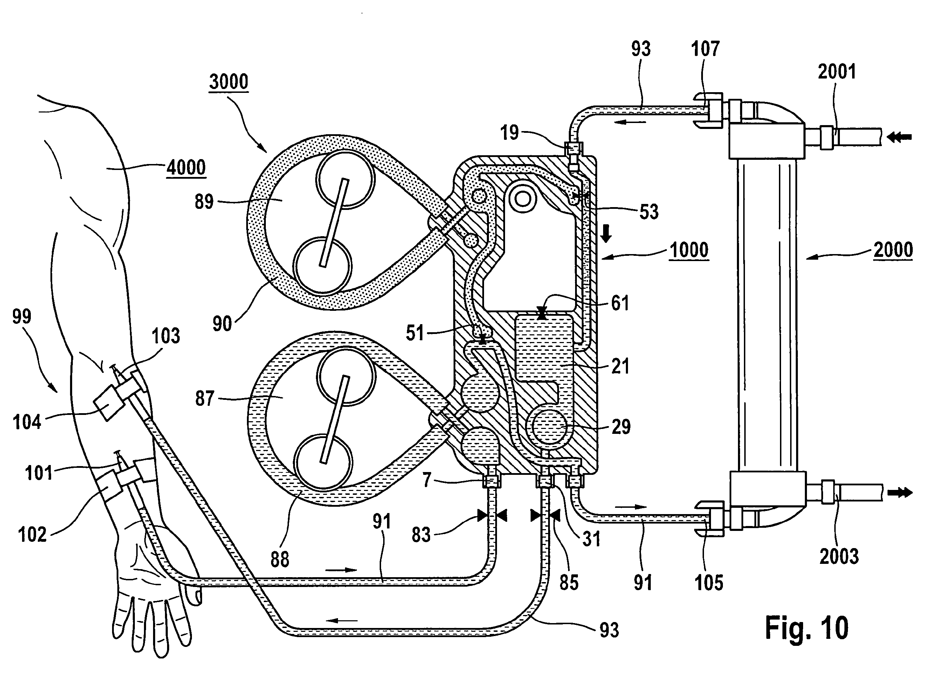

**Fig. 10** zeigt eine Phase des anhand von Fig. 8 dargestellten Blutbehandlungsprozess unter Verwenden einer Postdilution des Bluts mit Substituat ("Online HDF Postdilution").

Das Postdilutions-Zugabeventil 53 wird geöffnet Das Prädilutions-Zugabeventil 51 und das Single-Needle-Blutventil 61 sind geschlossen. Die Blutpumpe 87 wird betrieben. Die Substituatpumpe 89 wird gestartet. Die Substituatpumpe 89 kann in Gleichtakt mit der Blutpumpe 87 betrieben werden.

Die beschriebene Konfiguration ist als Momentaufnahme in Fig. 10 gezeigt, wobei sich zwischen Prädilutions-Zugabeventil 51 und Postdilutions-Zugabeventil 53 Substituat befindet und der restliche extrakorporale Kreislauf 3000 von Blut durchströmt wird.

Die Substituatpumpe 89 fördert Substituat, welches am Postdilutions-Zugabeventil 53 in den venösen Abschnitt 93 des extrakorporalen Kreislaufs 3000 gelangt und sich mit dem gereinigten Blut vermischt.

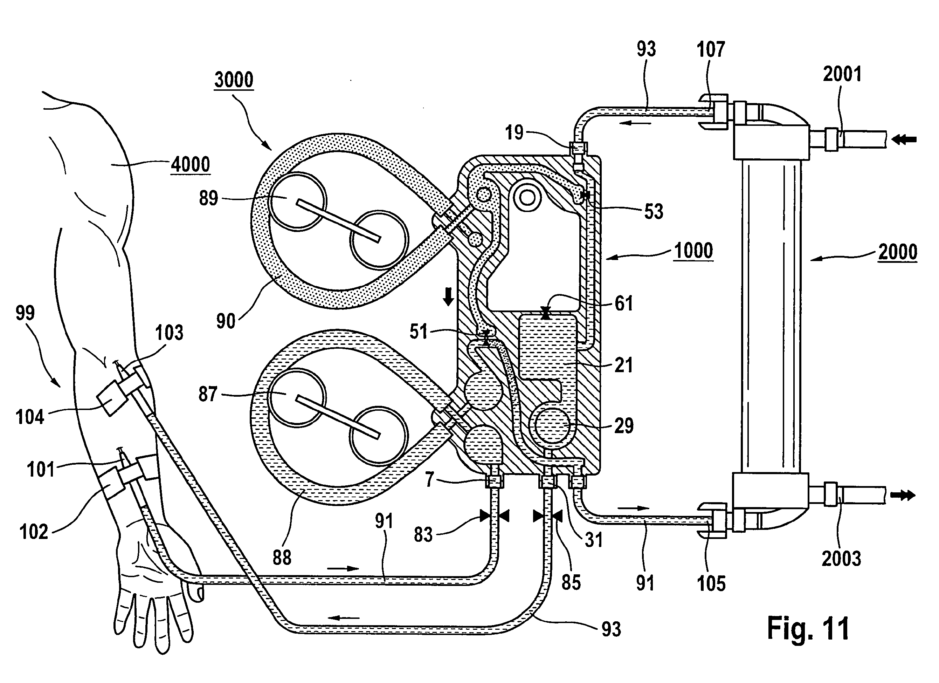

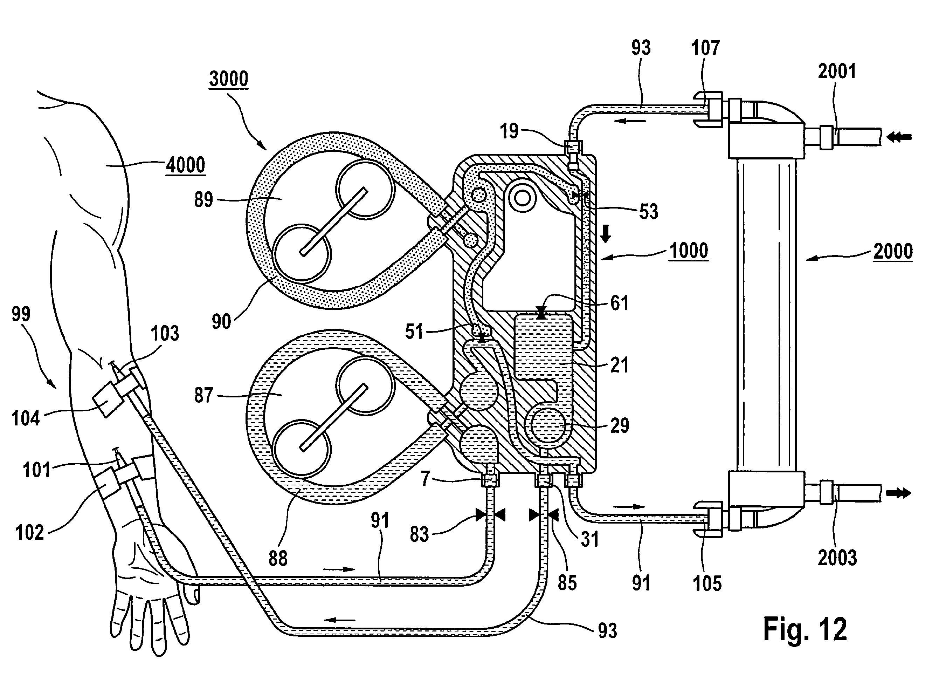

**Fig. 11** und **Fig. 12** zeigen Phasen des anhand von Fig. 8 dargestellten Blutbehandlungsprozess unter Verwenden einer Mischdilution des Bluts mit Substituat ("Online HDF Mischdilution - switched"). Der Begriff "Mischdilution" bezeichnet eine abwechselnd aus Prädilution und Postdilution stattfindende Verdünnung des Bluts mit Substituat.

**Fig. 11** veranschaulicht das Prädilutions-Intervall der Mischdilution anhand einer Momentaufnahme, **Fig. 12** das Postdilutions-Intervall der Mischdilution anhand einer Momentaufnahme.

Der anhand von Fig. 11 gezeigte Prozess entspricht dabei demjenigen des anhand von Fig. 9 gezeigten Prozesses, der anhand von Fig. 12 gezeigte Prozess demjenigen aus Fig. 10.

Die Blutpumpe 87 und die Substituatpumpe 89 rotieren im Gleichtakt. Die Blutpumpe 87 und die Substituatpumpe 89 können beispielsweise schneller rotieren als in den in den Fig. 9 und 10 dargestellten Verfahren bzw. Verfahrensabschnitten.

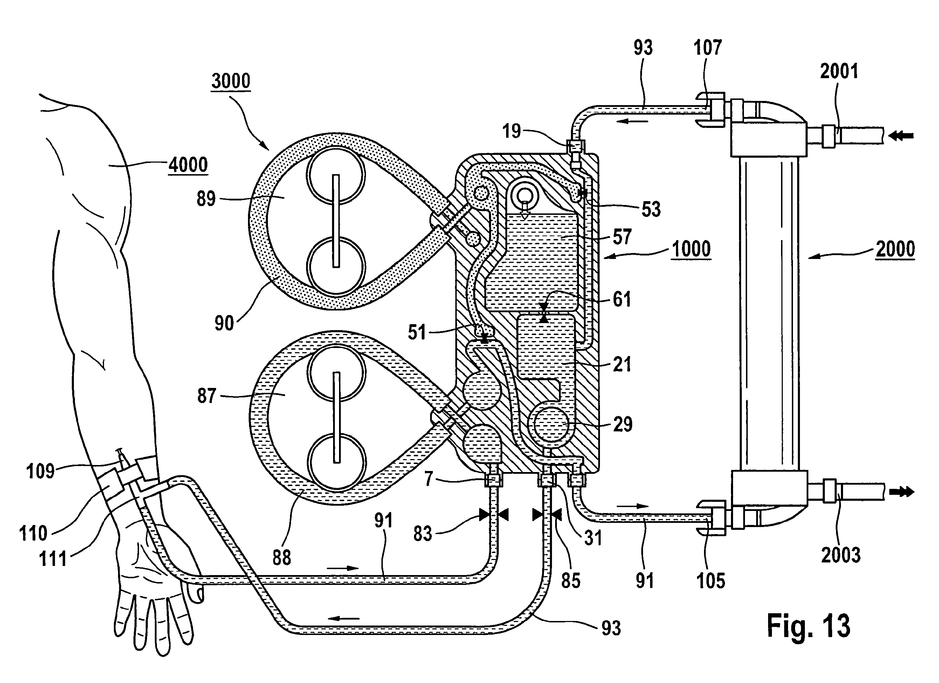

**Fig. 13** zeigt die Kassette 1000 und den extrakorporalen Kreislauf 3000 in einer Phase einer Dialysebehandlung mittels eines Single-Needle-Zugangs zum Patienten ("Cassette Integrated Single Needle").

Dem Patienten 4000 ist ein Single-Needle-Zugang 109 gelegt und mittels Fixierung 110 fixiert worden. Eine Fixierung 110 kann beispielsweise wiederum als Manschette, Pflaster oder dergleichen ausgestaltet sein.

Der Single-Needle-Zugang 109 weist ein Y-Stück bzw. eine Y-förmige Verzweigung 111 in den arteriellen Abschnitt 91 und den venösen Abschnitt 93 des extrakorporalen Kreislaufs 3000 auf.

Die arterielle Patientenschlauchklemme 83 wird geöffnet. Die venöse Patientenschlauchklemme 85 ist zunächst geschlossen. Dies ist in Fig. 13 nicht dargestellt.

Die Blutpumpe 87 wird gestartet. Die Dialysiereinrichtung 2000 wird betrieben. Durch den arteriellen Abschnitt 91 wird Blut vom Patienten 4000 in die Dialysiereinrichtung 2000 gefördert. In der Dialysiereinrichtung 2000 wird das Blut gereinigt. Das gereinigte Blut wird in den venösen Abschnitt 93 des extrakorporalen Kreislaufs 3000 eingebracht.

Das Blut tritt in die venöse Blutkammer 21 der Kassette 1000 ein. Das Single-Needle-Blutventil 61 ist geöffnet. Blut strömt in die Single-Needle-Kammer 57 ein.

Wenn die Single-Needle-Kammer 57 nahezu gefüllt ist, wird die Blutpumpe 87 gestoppt und die venöse Patientenschlauchklemme 85 wird geöffnet, wie in Fig. 13 zu erkennen ist. Der Dialysiervorgang wird gestoppt.

Das Blut wird schwerkraftbedingt aus der Single-Needle-Kammer 57, der venösen Blutkammer 21 und dem Gerinnselfänger 29 aus der Kassette 1000 ausgetragen und in den Patienten 4000 rückgeführt.

Wenn die Single-Needle-Kammer 29 nahezu von Blut geleert ist, wird die Blutpumpe 87 erneut gestartet.

Diese Phase der Blutbehandlung wird so oft wie erforderlich wiederholt.

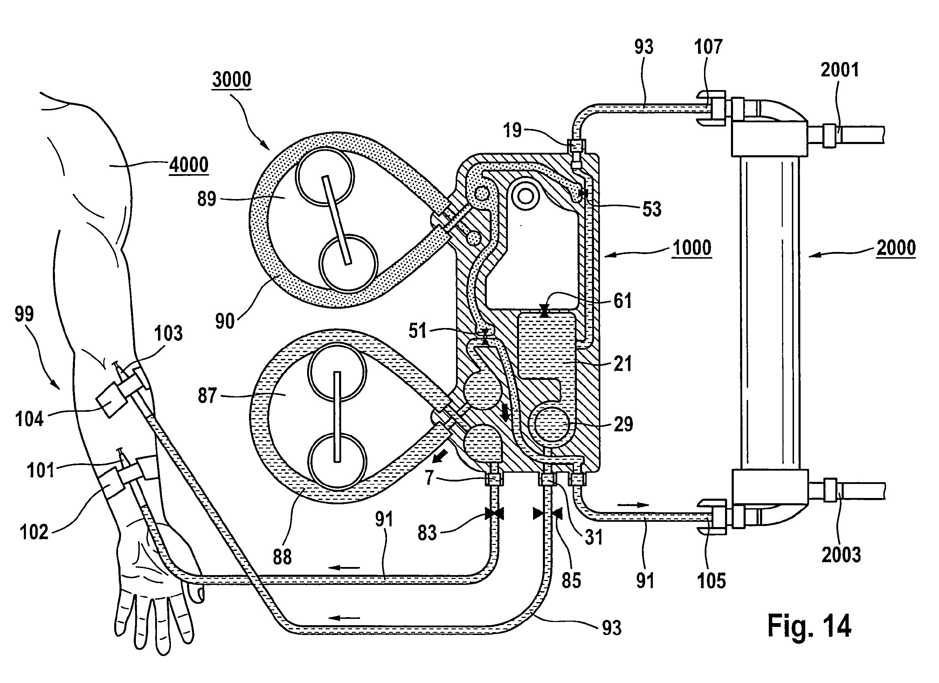

**Fig. 14** zeigt eine Phase einer ersten Variante eines Blutrückführungsprozesses nach Beendigung der Blutbehandlung.

Die arterielle Patientenschlauchklemme 83 ist geöffnet. Die venöse Patientenschlauchklemme 85 ist geschlossen. Die Substituatpumpe 89 wird betrieben.

Im extrakorporalen Kreislauf 3000 befindet sich Blut. In der Substituatleitung 49 befindet sich Substituat.

Das Prädilutions-Zugabeventil 51, das Postdilutions-Zugabeventil 53 und das Single-Needle-Blutventil 61 sind zunächst geschlossen.

Das Prädilutions-Zugabeventil 51 wird geöffnet. Substituat wird durch den arteriellen Abschnitt 91 des extrakorporalen Kreislaufs 3000 in Richtung der Dialysiereinrichtung 2000 gefördert.

Das im extrakorporalen Kreislauf 3000 in Förderrichtung hinter dem bzw. stromaufwärts des Prädilutions-Zugabeventils 51 befindliche Blut wird durch die Dialysiereinrichtung 2000 und den venösen Abschnitt 93 zum Patienten 4000 gefördert.

Kurz bevor das Substituat zur venösen Nadel 103 gelangt, wird die Substituatpumpe 89 gestoppt.

Die venöse Patientenschlauchklemme 85 wird geöffnet. Die arterielle Patientenschlauchklemme 83 wird geschlossen.

Fig. 14 zeigt die Konfiguration der Kassette 1000 in einer Momentaufnahme während des Prozesses, bei welcher das Substituat durch das geöffnete Prädilutions-Zugabeventil 51 stromaufwärts in den extrakorporalen Kreislauf 3000 eingebracht wird und das Blut verdrängt.

Die Blutpumpe 87 und die Substituatpumpe 89 werden betrieben. Die Blutpumpe 87 rotiert im Uhrzeigersinn und damit gegen die Förderrichtung. Die Substituatpumpe rotiert gegen den Uhrzeigersinn. Die Blutpumpe 87 und die Substituatpumpe 89 können zueinander versetzt rotieren.

Das Substituat wird vom Postdilutions-Zugabeventil 53 aus in den arteriellen Abschnitt 91 des extrakorporalen Kreislaufs 3000 und durch das Pumpschlauchsegment 88 der Blutpumpe 87 in Richtung des Patienten 4000 gefördert. Das Substituat verdrängt das im arteriellen Abschnitt 91 zwischen der arteriellen Nadel 101 und dem Prädilutions-Zugabeventil 51 befindliche Blut.

Kurz bevor das Substituat die arteriellen Nadel 101 erreicht, werden die Blutpumpe 87 und die Substituatpumpe 89 gestoppt.

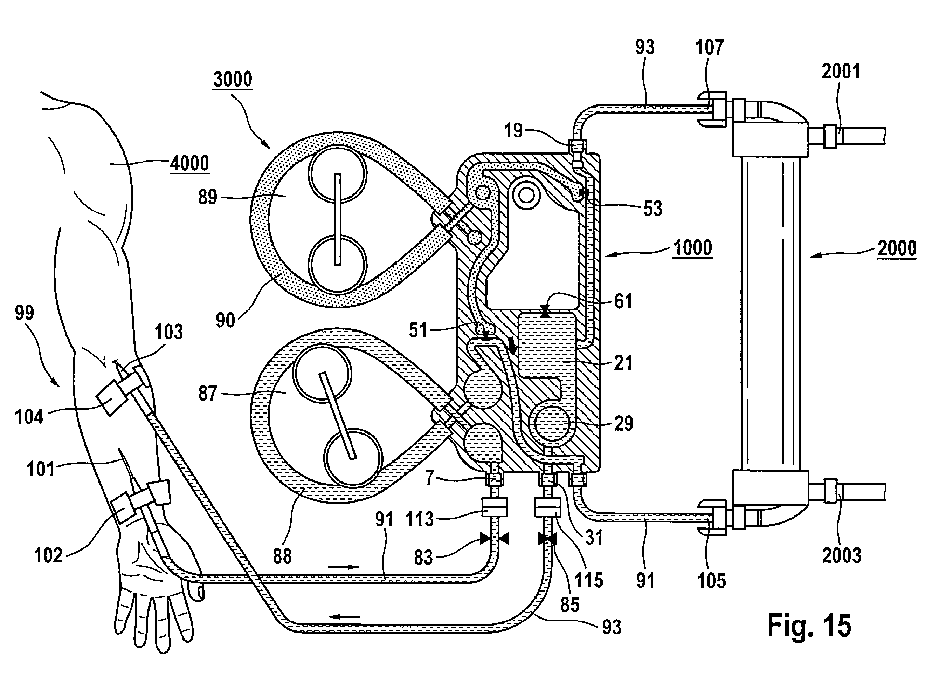

**Fig. 15** veranschaulicht eine Phase eines alternativen Blutrückführungsprozesses. An der arteriellen Patientenschlauchklemme 83 bzw. der venösen Patientenschlauchklemme 85 sind jeweils ein Sensor/Detektor 113 bzw. ein Sensor/Detektor 115 zum Messen der optischen Dichte des Leitungsinneren des extrakorporalen Kreislaufs 3000 und zum automatischen Erkennen des Auftretens von Substituat vorgesehen. Es können andere und/oder weitere geeignete Sensoren eingesetzt werden. Sensor und Detektor können als Einkomponentenbauteil oder als separate Komponenten realisiert sein.

Die Fixierung 102 der arteriellen Nadel 101 wird gelöst und die arterielle Nadel 101 wird gezogen. Die arterielle Patientenschlauchklemme 83 ist geöffnet. Das Prädilutions-Zugabeventil 51 ist geschlossen.

Die venöse Nadel 103 bleibt mit dem Patienten 4000 konnektiert. Die venöse Patientenschlauchklemme 85 ist geschlossen.

Die Blutpumpe 87 wird in Förderrichtung betrieben und fördert Blut aus dem arteriellen Abschnitt 91 des extrakorporalen Kreislaufs 3000.

Eine entsprechende Momentaufnahme des Prozesses, die einen Zustand kurz nach Beginn des Prozesses zeigt, ist in Fig. 15 gezeigt.

Der Sensor/Detektor 113 erkennt das Auftreten von Substituat. Das Blut wird weiter gefördert, bis es das Prädilutions-Zugabeventil 51 erreicht. Dann wird die Blutpumpe 87 gestoppt.

Die Substituatpumpe 89 wird gestartet. Die arterielle Patientenschlauchklemme 83 wird geschlossen. Die venöse Patientenschlauchklemme 85 wird geöffnet.

Das Prädilutions-Zugabeventil 51 wird geöffnet. Die Substituatpumpe 89 fördert das Substituat durch den arteriellen Abschnitt 91 des extrakorporalen Kreislaufs 3000, durch die Dialysiereinrichtung 2000 und den venösen Abschnitt 93 des extrakorporalen Kreislaufs 3000, bis der Sensor/Detektor 115 an der venösen Patientenschlauchklemme 85 das Auftreten von Substituat erkennt.

Das Blut wird durch die venöse Nadel 103 zum Patienten 4000 rückgefördert.

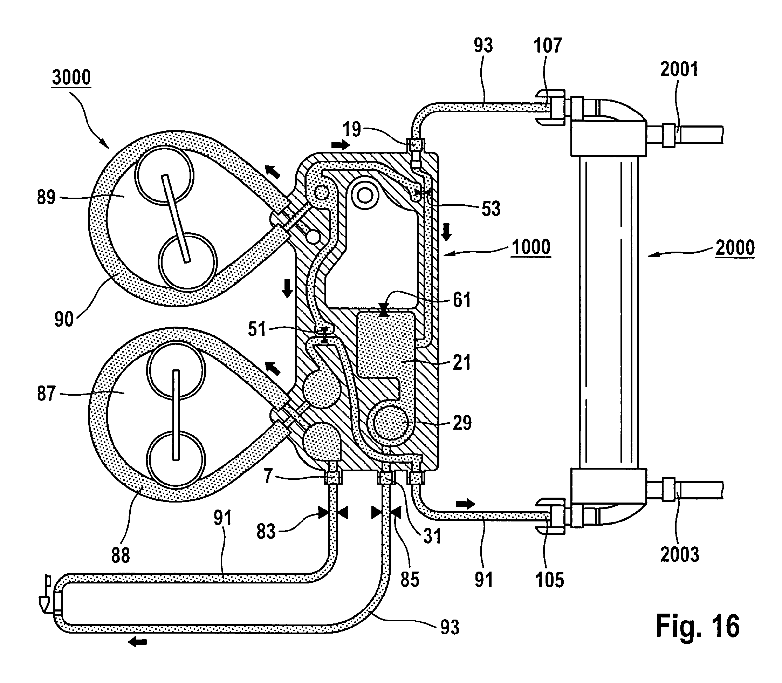

**Fig. 16** zeigt die Kassette 1000 und den extrakorporalen Kreislauf 3000 in einer Phase eines Entleerungs- oder Emptying-Prozesses.

Der Patient ist nicht mehr mit der Behandlungsvorrichtung konnektiert. Der arterielle Abschnitt 91 und der venöse Abschnitt 93 des extrakorporalen Kreislaufs 3000 sind miteinander verbunden.
Die arterielle Patientenschlauchklemme 83 und die venöse Patientenschlauchklemme 85 werden geöffnet. Das Prädilutions-Zugabeventil 51 und das Postdilutions-Zugabeventil 53 sind geöffnet, wie es in Fig. 16 in Form einer Momentaufnahme veranschaulicht ist.

Durch die Substituat-Zugabestelle 41 wird unter Betreiben der Substituatpumpe 89 Luft in den extrakorporalen Kreislauf 3000 eingetragen. Die Luft durchströmt das geöffnete Prädilutions-Zugabeventil 51 und das geöffnete Postdilutions-Zugabeventil 53 und gelangt so in den extrakorporalen Kreislauf 3000.

Die Luft durchströmt den extrakorporalen Kreislauf 3000 und die Dialysiereinrichtung 2000 in Gegenrichtung.

Das Prädilutions-Zugabeventil 51 wird geschlossen und die Blutpumpe 87 betrieben. Die Blutpumpe 87 und die Substituatpumpe 89 fördern die Luft in Förderrichtung durch die Kassette 1000 in die Dialysiereinrichtung 2000. Die Luft tritt am Dialysierflüssigkeitsaustritt 2003 aus.

Weitere geeignete Verfahren zum Rückführen des Bluts in einen Patienten und/oder zum Entleeren des extrakorporalen Kreislaufs sind beispielsweise in der Patentanmeldung 10 2009 008 346.4 10 2009 008 346.4 mit dem Titel "Verfahren zum Entfernen von *Blut aus einem extrakorporalen Blutkreislauf für eine Behandlungsvorrichtung nach Beenden einer Blutbehandlungssitzung und Vorrichtung zum Ausführen desselben"*, die am 11. Februar 2009 beim DPMA eingereicht wurde, beschrieben.

Derartige Verfahren sind ferner in der Patentanmeldung der vorliegenden Anmelderin 10 2009 024 606.1 mit dem Titel "*Verfahren zum Entfernen von Blut aus einem extrakorporalen Blutkreislauf, Behandlungsvorrichtung sowie Schlauchsystem*", die am 10. Juni 2009 beim DPMA eingereicht wurde, beschrieben.

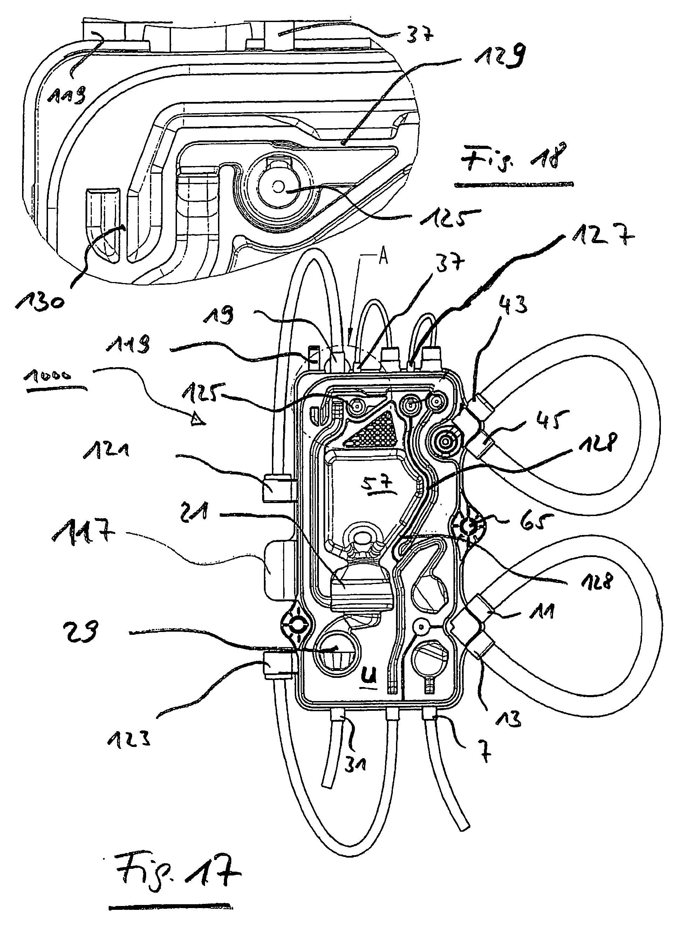

**Fig. 17** zeigt schematisch vereinfacht eine erfindungsgemäße externe Funktionseinrichtung 1000 in einer weiteren Ausführungsform, betrachtet von ihrer Vorderseite. Dabei entspricht die Kassette 1000 der Fig. 17 sowie aller folgenden Figuren in der überwiegenden Anzahl ihrer Merkmale der in den Figuren 1 bis 16 dargestellten Ausführungsform der Kassette 1000.

Die in Fig. 17 gezeigte Ausführungsform der Kassette 1000 weist einen Handgriff 117 zum - vorteilhaft einfachen und/oder raschen - Aufrüsten und/oder Abrüsten der Kassette 1000 vor oder nach Beendigen der Blutbehandlung auf. Der Handgriff 117 kann auch bei einem Abrüsten von Nutzen sein, bei welchem allgemein ein höherer Kraftaufwand erforderlich ist.

Der Handgriff kann ausgestaltet sein, um mittels Werkzeug, als nicht vornehmlich oder ausschließlich mit der Hand betätigt zu werden.

Ferner weist die in Fig. 17 gezeigte Ausführungsform der Kassette 1000 eine Zapfstelle 119 auf. Die Zapfstelle 119 der Kassette 1000 kann zum Entnehmen von Substituat aus der Kassette 1000 vorgesehen sein.

Die Zapfstelle 119 kann zusätzlich zu allen in den Fig. 1 bis 16 gezeigten Strukturen und Leitungen vorgesehen sein.

Die Zapfstelle 119 kann vorgesehen sein, um im normalen Blutbehandlungsbetrieb der Kassette 1000 nicht von Fluid durchströmt zu sein. Vorzugsweise verlässt in diesem Fall während der Behandlung kein Fluid die Kassette 1000 durch die Zapfstelle 119 oder tritt durch die Zapfstelle in die Kassette 1000 ein.

Die Zapfstelle 119 kann erfindungsgemäß auch an anderer Stelle als der in den Figuren dargestellten Stelle vorgesehen sein.

Die Zapfstelle 119 kann erfindungsgemäß beispielsweise in Ausnahmefällen als Stelle zum Entnehmen eines Fluids wie z. B. Substituat dienen. Das entnommene Fluid, z.B. Substituat, kann als Verdrängungsfluid bei einer, ggf. manuellen, Rückgabe oder arteriellen Infusion von extrakorporalem Blut in das Gefäßsystem des Patienten mittels der arteriellen Leitung verwendet werden. Letzteres kann im Falle einer Blockierung bestimmter Leitungen oder bei Versagen von Kassettenfunktionen oder von Funktionen der Behandlungsvorrichtung von Nutzen sein, indem über die Zapfstelle ohne besonderen Aufwand ein zum Zwecke des Rückführens geeignetes Fluid gewonnen werden kann.

Die Zapfstelle 119 kann auf verschiedene Weise an der Kassette 1000 vorgesehen und mit dieser verbunden sein. In bestimmten erfindungsgemäßen Ausführungsformen ist die Zapfstelle einstückig mit dem Gehäusekörper der Kassette 1000 hergestellt. Die Zapfstelle kann ein an die Kassette angespritzter Luer-Verschluss sein oder einen solchen aufweisen.

In manchen erfindungsgemäßen Ausführungsformen weist die Zapfstelle ein Ventil, insbesondere ein schaltbares Ventil, auf.

In bestimmten erfindungsgemäßen Ausführungsformen ist die Zapfstelle auch zur Zugabe eines Fluids in das Substituat geeignet und vorgesehen.

Die externe Funktionseinrichtung 1000 der Fig. 17 weist ferner Schlauchfixierungen 121 und 123 auf. Die Schlauchfixierungen 121, 123, welche auch in beliebiger anderer Anzahl vorgesehen sein können, können in bestimmten Ausführungsformen vorteilhaft verhindern, dass die bei Einsatz der Kassette 1000 erforderlichen Schläuche bei der Handhabung der Kassette 1000 stören oder beschädigt werden können, beispielsweise indem sie beim Ankoppeln der Kassette 1000 z.B. in die Öffnung einer Tür der Behandlungsvorrichtung hineinhängen.

Mit dem Bezugszeichen 125 ist ein Rückschlagventil bezeichnet, welches beispielsweise so ausgestaltet ist wie in der Patentanmeldung 10 2009 024 469.7 der Anmelderin der vorliegenden Erfindung, mit dem Titel "*Ventilvorrichtung, Ventileinsatz, externe Funktionseinrichtung, Behandlungsvorrichtung sowie Verfahren*", die am 10. Juni 2009 beim DPMA eingereicht wurde, offenbart.

Das Rückschlagventil 125 kann ein Zugabeventil einer venösen Leitung sein.

Fig. 17 offenbart ferner eine Heparinzugabestelle 127. Diese ist im Ausführungsbeispiel der Fig. 17 in einem oberen Bereich der Kassette 1000 oberhalb der Single-Needle-Kammer 57 und oberhalb der venösen Blutkammer 21 angeordnet.

Die Heparinzugabestelle 127 steht in Fluidverbindung mit einer Zufuhrleitung für Heparin, kurz Heparinleitung 128. Diese erstreckt sich in der Ausführungsform der Fig. 17 von der Heparinzugabestelle 127 hinunter bis auf eine Höhe der Kassette 1000, in welcher die venösen Blutkammer 21 beginnt.

Gut zu erkennen ist in Fig. 17 ferner, dass die Kassette 1000 in einem unteren Randbereich U, z.B. in Nachbarschaft des Gerinnselfängers 29 oder auch darunter, vergleichsweise wenige Strukturen, welche Funktionen übernehmen, aufweist. Damit ist auf der Kassette 1000 im Randbereich U Platz geschaffen oder gelassen worden, um die Ankoppelung der Kassette 1000 mit Messeinrichtungen oder dergleichen zu ermöglichen, welche z.B. mit der Behandlungsvorrichtung, einer Tür hiervon oder anderen Einrichtungen, verbunden sind. Derartige Messvorrichtungen können arterielle und/oder venöse Temperatursensoren sein, Air-bubble-Detektoren, Sensoren zum Messen einer optischen Dichte, und dergleichen.

Das Vorsehen des hierzu erforderlichen Platzes mit der hiermit verbundenen Möglichkeit, diesen mit den oben genannten und anderen Sensoren zu belegen, dient somit der Verfahrenssicherheit und damit dem Ausschluss von Risiken für den Patienten.

Es ist offensichtlich, dass der Bereich U auch in einem anderen Bereich der Kassette 1000 vorgesehen sein kann. Ebenso können mehr als nur ein solcher Bereich vorgesehen sein. Besonders bevorzugt sind solche Bereiche in einem Randbereich der Kassette 1000 und/oder an oder in der Nähe von zu- oder ableitenden Leitungen vorgesehen. Aufgrund derart günstiger Lagen möglicher Sensoren dient der Bereich direkt oder indirekt auch der Erhöhung der Zugänglichkeit, der Verbesserung der Ergonomie sowie dem Senken von Kosten z.B. für die Anbindung der vorgenannten Sensoren mit Auswerteeinheiten (kurze Signalleitungen).

**Fig. 18** zeigt schematisch vereinfacht eine vergrößerte Darstellung eines Bereichs A der Fig. 17. Das in Fig. 18 vergrößert dargestellte Rückschlagventil 125 kann z.B. als Ventil für die Postdilutionszugabe von Substituat derart in die Kassette 1000 integriert sein, dass es nach Beendigung der Behandlung mit Substituat umspült werden kann. Auf diese Weise kann es vorteilhaft möglich sein, dass Kavitäten beim Primen und Spülen der Kassette 1000 im Bereich des Rückschlagventils effektiver entlüftet werden können. Es kann vorteilhaft möglich sein, dass durch diese Anordnung nach dem Entleeren der Kassette 1000 nach Beendigung der Blutbehandlung keine oder keine sichtbaren Blutrückstände verbleiben. Auf diese Weise kann unter Umständen auch zu einer Verringerung der Kontaminationsgefahr Dritter beim Entsorgen der Kassette 1000 nach Gebrauch durch darin verbliebenes Blut beigetragen werden. Das Rückschlagventil 125 ist vorzugsweise derart angeordnet, um insbesondere im üblichen Gebrauch der Kassette 1000 ohne menschliches Zutun umspült zu werden.

Fig. 18 zeigt ferner ein Phantomventil 129 sowie ein Phantomventil 130. Phantomventile sind an anderen Stellen dieser Anmeldung ausführlich beschrieben. Für Details hierzu wird auf diese Stellen verwiesen.

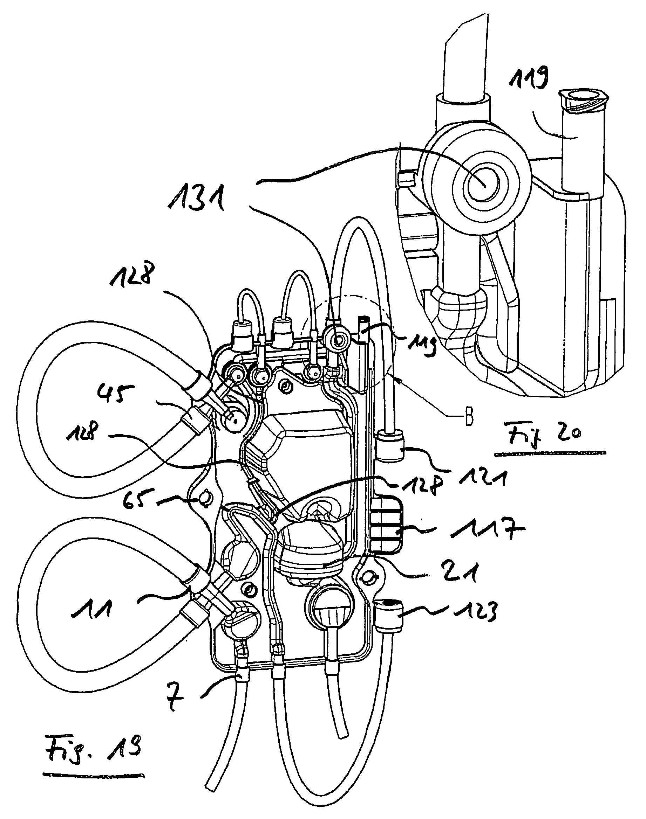

**Fig. 19** zeigt schematisch vereinfacht die erfindungsgemäße externe Funktionseinrichtung der weiteren Ausführungsform, dargestellt in einer leicht perspektivischen Ansicht mit Blick im Wesentlichen auf ihre Rückseite.

Neben den oben zu anderen Figuren bereits besprochenen Strukturen weist die in Fig. 19 gezeigte Ausführungsform der Kassette 1000 ferner eine Zugabestelle 131 auf, welche ein Septum aufweist. Die Zugabestelle 131 mit Septum (auch kurz Septumzugabestelle genannt) ist in der in Fig. 19 gezeigten Ausführungsform auf dem Niveau des oberen Randes der Kassette 1000 angeordnet. Der obere Rand der Fig. 19 entspricht einem Beispiel eines oberen Bereichs der Kassette 1000.

**Fig. 20** zeigt schematisch vereinfacht den Bereich B der Darstellung der Fig. 19 in Vergrößerung.

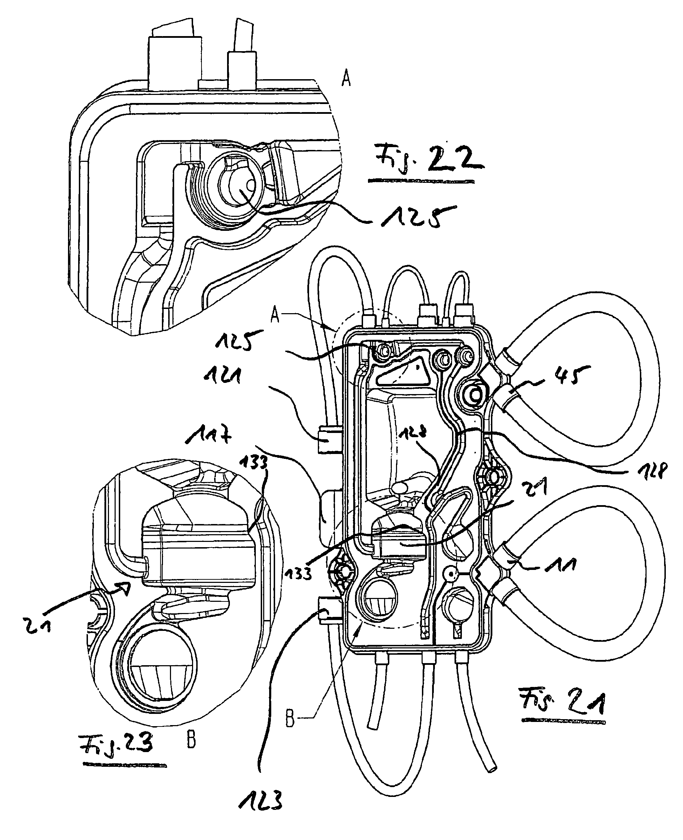

**Fig. 21** zeigt schematisch vereinfacht die erfindungsgemäße externe Funktionseinrichtung in einer wiederum weiteren Ausführungsfonn in einer leicht perspektivischen Ansicht, im Wesentlichen von ihrer Vorderseite.

In dieser wiederum weiteren Ausführungsform weist die Kassette 1000 anders als in der Ausführungsform z.B. der Fig. 17 keine Zapfstelle (dort mit Bezugszeichen 119 markiert) auf.

**Fig. 22** zeigt schematisch vereinfacht den Bereich A der Darstellung der Fig. 21 in Vergrößerung. Gut zu erkennen ist eine Anordnung des Phantomventils, welche sich von der Anordnung in Fig. 18 unterscheidet. Die unterschiedliche Anordnung kann zu unterschiedlicher Umspülbarkeit führen.

**Fig. 23** zeigt schematisch vereinfacht weitere Details, nämlich des Bereichs B, der Darstellung der Fig. 21.

Wie in Fig. 23 zu erkennen ist, weist die venöse Blutkammer 21 eine Einbuchtung 133 oder Einschnürung oder Neigungsveränderung oder Verjüngung oder Asymmetrie der inneren und/oder äußeren Wandung der venösen Blutkammer 21 auf. Die Einbuchtung 133 ist in Fig. 21 und Fig. 23 jeweils am rechten Rand und der Vorderseite der venösen Blutkammer 21 zu erkennen.

Die Einbuchtung 133 kann sich zumindest auf der der Zuströmleitung gegenüber liegenden Seite befinden.

Die Einbuchtung 133 kann einen Abschnitt des Umfangs oder den gesamten Umfang des Hartteils der venösen Kammer betreffen.

Die Einbuchtung 133 kann im Wesentlichen horizontal liegen in einer Gebrauchsstellung der Kassette 1000.

Die Einbuchtung 133 kann in bestimmten Ausführungsformen einer Veränderung des Umfangs und/oder des Durchmessers eines Abschnitts der venösen Blutkammer 21 oder der Wandung hiervon entsprechen oder diese umfassen.

Die Einbuchtung 133 kann in manchen erfindungsgemäßen Ausführungsformen einen nicht-halbkreisrunden Durchmesser der venösen Blutkammer 21 (in Höhe der Einbuchtung 133) in einem horizontalen Schnitt (bezogen auf die Darstellung der Kassette 1000 in Fig. 21 oder auf eine Anordnung der Kassette 1000 bei ihrem Gebrauch) bedeuten oder diesen umfassen.

Die Einbuchtung 133 kann in bestimmten erfindungsgemäßen Ausführungsformen eine Verjüngung des Kammerquerschnitts sein, insbesondere in der oben-unten-Richtung der Darstellung der Fig. 21.

Die Einbuchtung 133 kann in manchen erfindungsgemäßen Ausführungsformen ein Abschnitt oder Übergangsbereich sein, in oder mittels welchem ein größerer Querschnitt oder eine größere Querschnittsfläche der venösen Blutkammer 21 zu einem kleineren Querschnitt oder einer kleineren Querschnittsfläche der venösen Blutkammer 21 übergeht, insbesondere in der oben-unten-Richtung der Darstellung der Fig. 21.

Die Einbuchtung 133 kann in bestimmten erfindungsgemäßen Ausführungsformen eine Einbeulung sein, welcher sich über einen Bereich des Umfangs der venösen Blutkammer 21 erstreckt, insbesondere in der oben-unten-Richtung der Darstellung der Fig. 21.

Die Einbuchtung 133 kann in bestimmten Ausführungsformen zu einer asymmetrischen Form der venösen Blutkammer 21 gemessen an der Darstellung der Fig. 21 in oben-unten-Richtung führen.

Die Einbuchtung 133, welche teilweise oder vollständig über den gesamten Querschnitt der venösen Blutkammer 21 verlaufen kann, hat überraschender Weise zu einer verrinderten Schaumbildung innerhalb der venösen Blutkammer 21 geführt.

Bei der mittels der Einbuchtung 133 erzielten Optimierung der Geometrie der venösen Blutkammer 21, mit welcher optional auch die Geometrie eines Single-Needle-Ventils optimiert worden sein kann, kann in bestimmten erfindungsgemäßen Ausführungsformen eine verbesserte Entlüftung vor Behandlungsbeginn erzielt werden. Ferner kann in manchen erfindungsgemäßen Ausführungsformen eine verbesserte Dampfströmung beim Sterilisieren erzielt werden. Zudem kann in bestimmten erfindungsgemäßen Ausführungsformen eine Verringerung von Totwasser-Gebieten mit den hiermit verbundenen, bekannten Vorteilen erzielt werden.

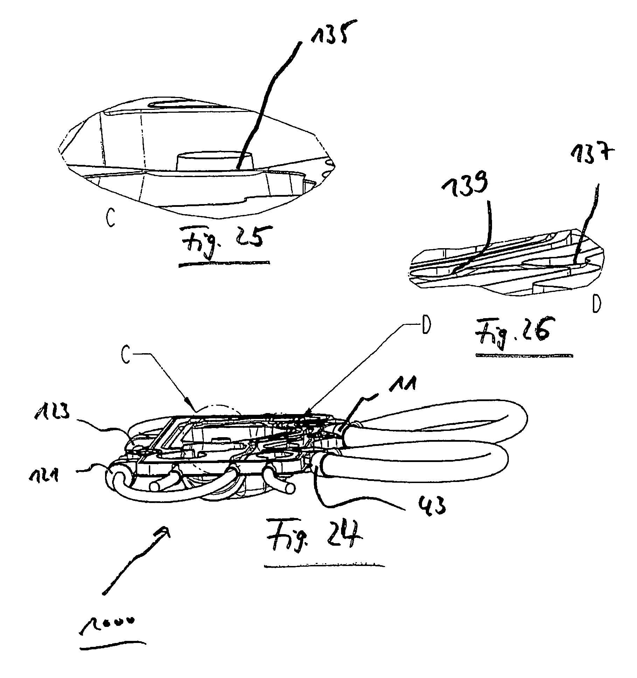

**Fig. 24** zeigt schematisch vereinfacht die erfindungsgemäße externe Funktionseinrichtung als Kassette 1000 in einer Perspektive schräg von unten und von vorne.

**Fig. 25** zeigt schematisch vereinfacht den Bereich C der Darstellung der Fig. 24 in Vergrößerung. Zu erkennen ist, dass ein Ventilsitz 135 des Single-Needle-(SN)-Ventils gegenüber der Umgebung oder benachbarten Stegen oder Teilen des Gehäusekörpers der Kassette 1000 abgesenkt ist. Die Erfinder konnten hierdurch strömungstechnische Vorteile erzielen, welche nicht zuletzt mit einer Vermeidung oder Verringerung der Ausbildung von Turbulenzen einhergehen können. Die Absenkung kann z.B. 0,5 mm betragen.

**Fig. 26** zeigt schematisch vereinfacht den Bereich D der Darstellung der Fig. 24 in Vergrößerung. Fig. 26 ist zu entnehmen, dass Ventilsitze 137 und 139 von Phantomventilen gegenüber der Umgebung oder benachbarten Stegen oder Teilen des Gehäusekörpers der Kassette 1000 abgesenkt sind. Die Absenkung kann z.B. 0,5 mm betragen.

## Patentansprüche

1. Externe Funktionseinrichtung, welche aufweist:
wenigstens einen Gehäusekörper,
wenigstens eine in den Gehäusekörper integrierte Kammer (9, 15, 21, 57) zum Aufnehmen medizinischer Fluide;
wenigstens einen in den Gehäusekörper integrierten Kanal (17, 19, 49) zum Aufnehmen und/oder Führen eines medizinischen Fluids; und
wenigstens eine in den Gehäusekörper, ganz oder teilweise integrierte Ventileinrichtung (35, 36, 39, 40, 47, 51, 53, 61), zum Steuern oder Regeln eines die externe Funktionseinrichtung durchströmenden Fluids,
wobei die externe Funktionseinrichtung an wenigstens einer ihrer Oberflächen mit einer Abdeckungseinrichtung versehen ist, welche Teil wenigstens der integrierten Ventileinrichtung (35, 36, 39, 40, 47, 51, 53, 61) ist, und
wobei die Abdeckungseinrichtung wenigstens in einem Abschnitt mit dem Gehäusekörper kraft-, form- und/oder stoffschlüssig verbunden ist,
wobei die externe Funktionseinrichtung als Kassette (1000) zu einer Blutbehandlung ausgestaltet ist, und
wobei die externe Funktionseinrichtung wenigstens eine venöse Blutkammer (21) aufweist,
wobei die wenigstens eine Ventileinrichtung ein Single-Needle-Blutventil (61) aufweist, welches zwischen der venösen Blutkammer (21) und einer Single-Needle-Kammer (57) der externen Funktionseinrichtung angeordnet ist, welche oberhalb, bezogen auf die Ausrichtung der externen Funktionseinrichtung während ihrer Verwendung, der venösen Blutkammer (21) angeordnet ist.

2. Externe Funktionseinrichtung nach Anspruch 1, wobei die Abdeckungseinrichtung mittels wenigstens einer umlaufenden Schweißnaht (5) mit dem Gehäusekörper verbunden ist.

3. Externe Funktionseinrichtung nach Anspruch 1 oder 2, wobei die Abdeckungseinrichtung mittels weiterer nicht-umlaufender, punktförmiger oder lokaler Verschweißungen mit dem Gehäusekörper verbunden ist.

4. Externe Funktionseinrichtung nach einem der Ansprüche 1 bis 3, wobei die Abdeckungseinrichtung wenigstens an zwei Seiten oder beidseits mit wenigstens einer Struktur der externen Funktionseinrichtung, verbunden ist.

5. Externe Funktionseinrichtung nach einem der Ansprüche 1 bis 4, wobei die Abdeckungseinrichtung eine Folie (3) ist.

6. Externe Funktionseinrichtung nach einem der vorangegangenen Ansprüche, welche Anschlüsse zu ihren Verbindungen mit einem extrakorporalen Kreislauf in Fluidkommunikation und zusätzlich wenigstens eine Zugabestelle (131) mit wenigstens einem Septum aufweist, wobei das Septum zum Zuführen eines Fluids, bei welchem es sich nicht um Blut oder nicht ausschließlich um Blut handelt, in ein Inneres oder einen Leitungsabschnitt der externen Funktionseinrichtung geeignet und vorgesehen ist.

7. Externe Funktionseinrichtung nach einem der vorangegangenen Ansprüche, aufweisend:
- eine Zufuhrleitung, welche ganz oder teilweise in einem oberen Bereich bezogen auf die Ausrichtung während ihres Gebrauchs oder einem Randbereich der externen Funktionseinrichtung angeordnet ist, und
- wenigstens eine Heparinleitung (128), welche wenigstens in einem Bereich der externen Funktionseinrichtung liegt, welche bei Gebrauch der externen Funktionseinrichtung in einem oberen Bereich hiervon liegt.

8. Externe Funktionseinrichtung nach einem der vorangegangenen Ansprüche, welche wenigstens einen Griff oder Handgriff (117) zum Verbinden und/oder Trennen der externen Funktionseinrichtung mit einer Behandlungseinrichtung oder von einer Behandlungseinrichtung aufweist.

9. Externe Funktionseinrichtung nach Anspruch 1, welche in einem hierin enthaltenen Abschnitt eines extrakorporalen Kreislaufs wenigstens eine arterielle kassettenintegrierte Kammer und wenigstens eine venöse kassettenintegrierte Kammer aufweist,
- wobei die Kassette (1000) wenigstens die Abdeckungseinrichtung, welche als eine Folie (3) ausgebildet ist, aufweist, und wobei über die Folie (3) ein arterieller und/oder ein venöser Druck, welcher im extrakorporalen Blutkreislauf herrscht, messbar ist, und
- wobei die externe Funktionseinrichtung eine Einrichtung zum Messen des arteriellen Drucks über der arteriellen Kammer und/oder zum Messen des venösen Drucks über der venösen Kammer über die Folie (3) aufweist.

10. Externe Funktionseinrichtung nach einem der vorangegangenen Ansprüche, welche zwei Konnektoren (11, 13, 43, 45) zum Fluid-Verbinden mit wenigstens einer Rollenpumpe aufweist, und wobei die externe Funktionseinrichtung mit wenigstens einem Pumpschlauchsegment (88, 90) ausgestaltet ist.

11. Externe Funktionseinrichtung nach einem der vorangegangenen Ansprüche, aufweisend:
- wenigstens eine Ventileinrichtung (36, 40, 51, 53, 61), welche wenigstens einen Steg, welcher am Gehäusekörper ausgestaltet ist, und wenigstens einen Abschnitt der Abdeckungseinrichtung aufweist,
- wobei Steg und Abdeckungseinrichtung angeordnet sind, um mittels eines mittels der Abdeckungseinrichtung auf einen Steg einwirkenden Aktors einer Blutbehandlungsvorrichtung zur Veränderung eines Fluiddurchflusses betätigbar zu sein.

12. Externe Funktionseinrichtung nach einem der vorangegangenen Ansprüche, welche ausgestaltet und vorgesehen ist, um mit der der Abdeckungseinrichtung zugewandten Oberfläche an eine Blutbehandlungsvorrichtung mittels einer Aufnahmeeinrichtung der Blutbehandlungsvorrichtung ankoppelbar zu sein.

13. Externe Funktionseinrichtung nach einem der vorangegangenen Ansprüche, aufweisend wenigstens eine Substituat-Zugabestelle (41), welche ein Berührschutzelement und/oder einen Tropfschutz aufweist.

14. Externe Funktionseinrichtung nach Anspruch 1, wobei die venöse Blutkammer (21) mittels einer Querschnittsveijüngung (27) des Gehäusekörpers in wenigstens einen oberen Raum (23) und wenigstens einen unteren Raum (25) aufgeteilt ist.

15. Externe Funktionseinrichtung nach Anspruch 14, wobei der obere Raum (23) und der untere Raum (25) in Fluidkommunikation oder in Fluidverbindung miteinander stehen und
- der obere Raum (23) ausgestaltet ist, um eine tangentiale Zuströmung von die externe Funktionseinrichtung durchströmenden Fluiden zuzulassen oder zu erzeugen, wobei der obere Raum (23) einen Bereich aufweist zum Erzeugen einer stabilen Rotationsströmung der die externe Funktionseinrichtung durchströmenden Fluide und
- wobei der untere Raum (25) einen Bereich aufweist, der im Wesentlichen oder vollständig frei von Rotationsströmung der die externe Funktionseinrichtung durchströmenden Fluide ist.

16. Externe Funktionseinrichtung nach einem der vorangegangenen Ansprüche, mit einem Originalitätsschutz.

17. Externe Funktionseinrichtung nach einem der vorangegangenen Ansprüche, mit einem Wiederverwendungsschutz.

18. System mit einer Blutbehandlungsvorrichtung und mit wenigstens einer externen Funktionseinrichtung gemäß einem der Ansprüche 1 bis 17, wobei die Blutbehandlungsvorrichtung wenigstens eine Steuereinrichtung, Aktoren und/oder Sensoren zum Ansteuern und/oder Betätigen der externen Funktionseinrichtung aufweist, und eine Aufnahmeeinrichtung zum Aufnehmen der externen Funktionseinrichtung, wobei die Aufnahmeeinrichtung eine Ankoppelfläche zum Ankoppeln der externen Funktionseinrichtung aufweist.

19. System nach Anspruch 18, wobei die Blutbehandlungsvorrichtung als Dialysevorrichtung ausgestaltet ist.

## Claims

1. An external functional means, comprising:
at least one housing body,
at least one chamber (9, 15, 21, 57) integrated into the housing body for receiving medical fluids;
at least one passage (17, 19, 49) integrated into the housing body for receiving and/or conducting a medical fluid; and
at least one valve means (35, 36, 39, 40, 47, 51, 53, 61) completely or partly integrated into the housing body for controlling or regulating a fluid flowing through the external functional means,
wherein the external functional means is provided on at least one of its surfaces with a cover means that is part of at least one integrated valve means (35, 36, 39, 40, 47, 51, 53, 61), and
wherein the cover means is connected by frictional and/or by form closure and/or by material connection to the housing body in at least one portion thereof,
wherein the external functional means is configured as a cassette (1000) for a blood treatment, and
wherein the external functional means comprises at least one venous blood chamber (21),
wherein the at least one valve means comprises a single-needle blood valve (61) arranged between the venous blood chamber (21) and a single-needle chamber (57) of the external functional means which is arranged above the venous blood chamber (21) with regard to the alignement of the external functional means during a state of use of the external functional means.

2. The external functional means according to claim 1, wherein the cover means is connected to the housing body by means of at least one peripheral weld (5).

3. The external functional means according to claim 1 or 2, wherein the cover means is connected to the housing body by means of additional, non-peripheral or dot-shaped or local welds.

4. The external functional means according to any one of claims 1 to 3 wherein the cover means is connected at at two sides or bilaterally with at least one structure of the external functional means.

5. The external functional means according to any one of claims 1 to 4, wherein the cover means is a film (3).

6. The external functional means according to any one of the preceding claims, which comprises connections for being connected in fluid communication to an extracorporeal circuit, and which is additionally comprising at least one addition site (131) comprising at least one septum suited and provided for supplying a fluid which is not or not exclusively blood, into an interior or into a line section of the external functional means.

7. The external functional means according to any one of the preceding claims comprising:
a supplying line which is completely or partly arranged in an upper area or in a border area of the external functional means with respect to the alignment of the external functional means during its use, and
at least one heparin line (128) arranged at least in an area of the external functional means which is arranged in an upper area thereof during the use of the external functional means.

8. The external functional means according to any one of the preceding claims comprising at least one handle bar or handhold (117) for connecting and/or separating the external functional means with or from a treatment apparatus.

9. The external functional means according to claim 1, which comprises, in a portion of an extracorporeal circuit (3000) contained therein, at least one arterial cassette-integrated chamber and at least one venous cassette-integrated chamber,
wherein the cassette (1000) comprises at least one cover means as a film (3), and wherein an arterial and/or a venous pressure present in the extracorporeal blood circuit may be measured through the intermediary of the film (3), and
wherein the external functional means comprises a means for measuring the arterial and/or venous pressure above the arterial and/or venous chamber through the intermediary of the film (3).

10. The external functional means according to any one of the preceding claims comprising two connectors (11, 13, 43, 45) adapted to be connected in fluid communication with at least one peristaltic pump, and wherein the external functional means comprises or is configured for receiving at least one pump tube segment (88, 90).

11. The external functional means according to any one of the preceding claims, comprising:
at least one valve means (36, 40, 51, 53, 61), which comprises at least one bar formed on the housing body and at least one portion of the cover means,
wherein bar and cover means are disposed so as to be operable by means of an actor of a blood treatment apparatus acting on a bar by means of the cover means in order to alter a flow of fluid.

12. The external functional means according to any one of the preceding claims, which is intended and adapted to be coupleable to a blood treatment apparatus by means of a reception means of the blood treatment apparatus with a surface facing the cover means.

13. The external functional means according to any one of the preceding claims, comprising at least one substituate addition site (41) having a touch-protection element and/or a drip-protection element.

14. The external functional means according to claim 1, wherein the venous blood chamber (21) is subdivided into at least one upper space (23) and at least one lower space (25) by means of a cross-sectional restriction (27) of the housing body.

15. The external functional means according to claim 14, wherein the upper space (23) and the lower space (25) are in fluid communication or in fluid connection with each other, and
the upper space (23) is configured for admitting or generating a tangential inflow of fluids flowing through the external functional means, wherein the upper space (23) includes a region for generating a stable rotational flow of the fluids flowing through the external functional means, and
wherein the lower space (25) includes a region that is substantially or entirely free from rotational flow of the fluids flowing through the external functional means.

16. The external functional means according to any one of the preceding claims, comprising a tamper protection.

17. The external functional means according to any one of the preceding claims, comprising a protection against reuse.

18. System with a blood treatment apparatus and at least one external functional means according to any one of claims 1 to 17, wherein the blood treatment apparatus comprises at least one control means, actors and/or sensors for driving and/or operating the external functional means, and a reception means for receiving the external functional means, wherein the reception means comprises a coupling surface for coupling the external functional means.

19. System according to claim 18 wherein the blood treatment apparatus is designed as a dialysis apparatus.

## Revendications

1. Un dispositif médical externe, comprenant:
au moins un corps de boîtier,
au moins une cavité (9, 15, 21, 57) pour recevoir des fluides médicaux intégrée dans le corps de boîtier;
au moins un passage (17, 19, 49) pour recevoir et/ou conduire le fluide médical intégré dans le corps de boîtier; et
au moins une vanne (35, 36, 39, 40, 47, 51, 53, 61) entièrement ou partiellement intégrée dans le corps de boîtier pour contrôler et/ou réguler un fluide s'écoulant au travers du dispositif médical,
où le dispositif médical externe est doté d'un moyen de revêtement sur l'une au moins de ses surface lequel fait part d'au moins une vanne intégrée (35, 36, 39, 40, 47, 51, 53, 61), et
où le moyen de revêtement est connecté dans l'une au moins de ses sections au corps de boîtier par adhérence, emboîtement mécanique et/ou liaison de matériau,
où le dispositif médical externe est configuré comme cassette (1000) pour un traitement du sang, et
où le dispositif médical externe comprend au moins une cavité veineuse pour le sang (21),
où l'une au moins des vannes comprend une vanne Single-Needle (61) agencée entre la cavité veineuse pour le sang (21) et une cavité Single-Needle (57) du dispositif médical externe qui est agencée en dessus de la cavité veineuse pour le sang (21) par rapport à l'orientation du dispositif médical externe lors de l'usage du dispositif médical externe.

2. Le dispositif médical externe selon la première revendication où le moyen de revêtement est connecté au corps de boîtier au moyen d'au moins une soudure périphérique (5).

3. Le dispositif médical externe selon la première ou la seconde revendication, où le moyen de revêtement est connecté au corps de boîtier au moyen de soudures additionnelles non-périphériques, ponctuelles ou locales.

4. Le dispositif médical externe selon l'une des revendications 1 à 3 où le moyen de revêtement est connecté au moins de deux côtés ou de façon bilatérale avec au moins une structure du dispositif médical externe.

5. Le dispositif médical externe selon l'une des revendications 1 à 4, où le moyen de revêtement est un film (3).

6. Le dispositif médical externe selon l'une des revendications précédentes, comprenant des connections connectables pour être en communication fluidique avec un circuit extracorporel sanguin, et comprenant de plus au moins un site d'addition (131) comprenant au moins un septum prévu et adapté pour fournir ou introduire un fluide n'étant pas ou pas exclusivement du sang, à l'intérieur d'un segment de ligne du dispositif médical externe.

7. Le dispositif médical externe selon l'une des revendications précédentes comprenant:
Une line de fourniture entièrement ou partiellement agencée dans une région supérieure ou limitrophe du dispositif médical externe par rapport à l'orientation du dispositif médical externe lors de son usage, et
au moins une ligne d'héparine (128) agencée au moins dans une région du dispositif médical externe qui est située, lors de l'usage de celui-ci, dans une région supérieure du dispositif médical externe.

8. Le dispositif médical externe selon l'une des revendications précédentes comprenant au moins un levier ou une poignée (117) pour connecter le dispositif médical externe avec un appareil de traitement et/ou pour séparer le dispositif médical externe d'un appareil de traitement.

9. Le dispositif médical externe selon la première revendication comprenant, dans une section d'un circuit extracorporel (3000) contenu dans le dispositif médical, au moins une cavité artérielle intégrée dans la cassette et au moins une cavité veineuse intégrée dans la cassette-,
où la cassette (1000) comprend au moins un moyen de revêtement comme film (3), et où une pression artérielle et/ou veineuse présente dans le circuit extracorporel sanguin peut être mesurée par l'intermédiaire du film (3), et
où le dispositif médical externe comprend des moyens pour mesurer la pression artérielle et/ou veineuse en dessus de la cavité artérielle et/ou veineuse par l'intermédiaire du film (3).

10. Le dispositif médical externe selon l'une des revendications précédentes comprenant deux connecteurs (11, 13, 43, 45) adaptés pour être connectés en communication fluidique avec au moins une pompe péristaltique, et où le dispositif médical externe comprend ou est configuré pour recevoir au moins un segment de tube de pompe.

11. Le dispositif médical externe selon l'une des revendications précédentes comprenant:
au moins une vanne (36, 40, 51, 53, 61), comprenant au moins un levier formé sur le corps de boîtier et au moins une portion du moyen de revêtement,
où le levier et le moyen de revêtement sont disposés de façon à être opérable au moyen d'un actionneur d'un appareil de traitement du sang agissant sur un levier au moyen du moyen de revêtement de façon à modifier l'écoulement du fluide.

12. Le dispositif médical externe selon l'une des revendications précédentes prévu et adapté pour être susceptible d'être raccordé à un appareil de traitement du sang au moyen d'un moyen de réception de l'appareil de traitement du sang avec une surface orientée en direction du moyen de revêtement.

13. Le dispositif médical externe selon l'une des revendications précédentes, comprenant au moins un site d'addition de substitut (41) ayant un élément de protection au toucher et/ou un élément de protection contre les gouttes d'eau.

14. Le dispositif médical externe selon la première revendication, où la cavité veineuse pour le sang (21) est divisée en au moins un espace supérieur (23) et au moins un espace inférieur (25) au moyen d'une restriction transversale (27) du corps de boîtier.

15. Le dispositif médical externe selon la revendication 14, où l'espace supérieur (23) et l'espace inférieur (25) sont en communication fluidique ou en liaison fluide l'une avec l'autre, et
L'espace supérieur (23) est configuré pour admettre ou générer une entrée tangentielle de fluides s'écoulant au travers du dispositif médical externe, où l'espace supérieur (23) inclut une région pour générer un flux rotationnel stable des fluides s'écoulant au travers du dispositif médical externe, et
Où l'espace inférieur (25) inclut une région dans laquelle les fluides s'écoulant au travers du dispositif médical externe sont substantiellement ou entièrement libres de flux rotationnel.

16. Le dispositif médical externe selon l'une des revendications précédentes, comprenant une protection contre les manipulations.

17. Le dispositif médical externe selon l'une des revendications précédentes, comprenant une protection contre la réutilisation.

18. Système avec un appareil de traitement du sang et au moins un dispositif médical externe selon l'une des revendication 1 à 17, où l'appareil de traitement du sang au moins un moyen de commande, des actionneurs et/ou des capteurs pour gérer et/ou faire fonctionner le dispositif médical externe, et un moyen de réception pour recevoir le dispositif médical externe, où le moyen de réception comprend une surface de couplage pour raccorder le dispositif médical externe.

19. Système selon la revendication 18 où l'appareil de traitement du sang est conçu comme appareil de dialyse.
